# EUROPEAN PATENT APPLICATION

(11) **EP 1 577 393 A1**
(43) Date of publication of application: **21.09.2005**
(21) Application number: 03778829.6
(22) Date of filing: 11.12.2003
(51) Int. Cl.: C12N 15/31, C12Q 1/68

(54) **PRIMER AND PROBE FOR DETECTING i VIBRIO CHOLERAE /i OR i VIBRIO MIMICUS /i AND DETECTION METHOD USING THE SAME**

(30) Priority: 13.12.2002 JP 2002362878
(71) Applicant: Nichirei Foods Inc., Tokyo 104-8402 (JP)
(72) Inventor: KOIZUMI, Takeshi, R&D Division, Nichirei Corp., Chiba-shi, Chiba 261-8545 (JP); NISHIYAMA, Yoko, R&D Division, Nichirei Corp., Chiba-shi, Chiba 261-8545 (JP); YAMAMOTO, Satoshi, R&D Division, Nichirei Corp., Chiba-shi, Chiba 261-8545 (JP); FUKUYAMA, Masafumi, College of Environ. Health, Sagamihara-shi, Kanagawa 229-8501 (JP); FURUHATA, Katsunori, College of Environ. Health, Sagamihara-shi, Kanagawa 229-8501 (JP); OONAKA, Kenji, College of Environ. Health, Sagamihara-shi, Kanagawa 229-8501 (JP)
(74) Representative: Woods, Geoffrey Corlett
(86) International application number: PCT/JP2003/015889
(87) International publication number: WO 2004/055188

(57) **Abstract**

To produce high-performance specific gene amplification primers for detecting, quantifying, or identifying *Vibrio cholerae* and *Vibrio mimicus,* having low risk of misidentification and practically sufficient amplification efficiency and amplification specificity. We have determined partial nucleotide sequences of *rpoD* and *gyrB* genes of *Vibrio cholerae, Vibrio mimicus,* and closely related species, revealed their phylogenetic relationship, and then identified nucleotides characteristic of *Vibrio cholerae* and *Vibrio mimicus,* respectively. Thus, we have made it possible to design probes having high specificity and gene amplification primers having high specificity and excellent amplification efficiency, both of which contain the characteristic nucleotides.

SEQUENCE LISTING

<110> Nichirei Corporation

<120> Primers and probes for detection of vibrio cholera or vibrio mimicus
and method of using thereof

<130> PH-1967-PCT

<140>
<141>

<150> JP 2002/362878
<151> 2002-12-13

<160> 6

<170> PatentIn Ver. 2.1

<210> 1
<211> 885
<212> DNA
<213> Artificial Sequence
<220>
<223> Description of Artificial Sequence: Consensus sequence of vibrio
cholera and vibrio mimicus -gyrB

<400> 1

gtmtccggyg gtctrcacgg ggtaggtgtg tcggtrgtka aygcsctbtc wgaaaaagtg 60

ctrctbacca tytatcgygg yggcaaraty caywcscaaa cttaccatca yggtgtgcca 120

caagcaccgt tgkctgtrgt rggtgakacw gagcgtaccg gtactaccgt acgtttctgg 180

ccwagygcac aracytttac caatatcgaa ttycattacg acattytggc taaacgyctg 240

cgtgagctgt cattcctgaa ytctggcgtg tcgatcaagc tgaysgatga rcgtgaagaa 300

gataaraaag accacttyat gtatgaaggk ggtattcaag cgtttgtkac ccacttgaac 360

cgyaayaaaa cgccratcca tgaraaagtm ttccacttya accaagagcg tgaagatggc 420

atcagcgtgg aagtggcrat gcagtggaay gatggtttcc aagaaaacat ctactgcttt 480

acyaacaaca tyccacagcg tgatggyggt acccayttag cyggtttccg tggtgcrttg 540

acccgtactt tgaacaacta yatggayaaa gaaggcttct cgaagaaagc scaagcrgca 600

acctcgggtg atgatgcgcg tgaaggctta acrgcdgtkg tdtcggtgaa agtrccrgat 660

cctaaattct cragccaaac caaagataag ctrgtttctt cggargtraa atccgcrgtt 720

gartcagcya tgaatgagaa gctggcrgat ttcctrgcgg aaaacccaag cgaagcgaaa 780

aacgtttgtt cgaagattat tgatgcrgcr cghgckcgtg aagcvgcgcg taaagcmcgk 840

gaaatgacyc gycgtaaagg cgcgytrgay ythgcwggyt trcch 885

<210> 2

<211> 822

<212> DNA

<213> Artificial Sequence

<220>

<223> Description of Artificial Sequence: Consensus sequence of vibrio

cholera and vibrio mimicus -rpoD

<400> 2

acacgtgaag gygaaatcga tattgccaag cgcattgaag atggtattaa ccaagttcaa 60

agtgcgattg ctgagtatcc tggaaccatc ccwtayattc ttgarcagtt tgaymrkgtt 120

caggcmgaag arctacgtct sactgayctg atttcwggtt tcgttgaycc taacgacatg 180

gaaaccgaag cgccaacygc kactcacatc ggttcwgarc tytctgaagc sgatctcgck 240

gatgaagatg aygmkgtcgy sgargatgaa gacgargatg aagaygaaga yggcgacggt 300

gaaagyagcg acagcgaaga agaagtsggt atygaccctg arctsgctcg tgagaaattc 360

aatgaactgc gcggyaagtt ccaaaacctg caattagcgg ttaatgaatt tggtcgtgac 420

agtmaycaag cwtctgaagc ktcarrcytr gtrytggata tyttccgyga attccgycta 480

acaccaaarc aattygacca yttggttgaa actctgcgya cytcratgga tcgtgttcgy 540

acccaagarc gyttggtrat gaaagcvgtr gttgaagtcg cgaaratgcc raagaaatcr 600

ttyatygcyc trtttacagg caatgaatcg aatgargart ggctbgataa agtvctygct 660

tctgayaarc cttaygtasm raaagtmcgt gagcaagaag amgakatycg ccgytcaaty 720

caraaactdc aratgatcga rcargagacw tcactgtctg ttgarcgyat caaagacatc 780

agccgtcgta tgtcwatcgg tgargcraaa gctcgccgtg cg 822

<210> 3

<211> 822

<212> DNA

<213> Artificial Sequence

<220>

<223> Description of Artificial Sequence: Consensus sequence vibrio

cholera-gyrB

<400> 3

acacgtgaag gygaaatcga tattgccaag cgcattgaag atggtattaa ccaagttcaa 60

agtgcgattg ctgagtatcc tggaaccatc ccwtayattc ttgarcagtt tgaymrkgtt 120

caggcmgaag arctacgtct sactgayctg atttcwggtt tcgttgaycc taacgacatg 180

gaaaccgaag cgccaacygc kactcacatc ggttcwgarc tytctgaagc sgatctcgck 240

gatgaagatg aygmkgtcgy sgargatgaa gacgargatg aagaygaaga yggcgacggt 300

gaaagyagcg acagcgaaga agaagtsggt atygaccctg arctsgctcg tgagaaattc 360

aatgaactgc gcggyaagtt ccaaaacctg caattagcgg ttaatgaatt tggtcgtgac 420

agtmaycaag cwtctgaagc ktcarrcytr gtrytggata tyttccgyga attccgycta 480

acaccaaarc aattygacca yttggttgaa actctgcgya cytcratgga tcgtgttcgy 540

acccaagarc gyttggtrat gaaagcvgtr gttgaagtcg cgaaratgcc raagaaatcr 600

ttyatygcyc trtttacagg caatgaatcg aatgargart ggctbgataa agtvctygct 660

tctgayaarc cttaygtasm raaagtmcgt gagcaagaag amgakatycg ccgytcaaty 720

caraaactdc aratgatcga rcargagacw tcactgtctg ttgarcgyat caaagacatc 780

agccgtcgta tgtcwatcgg tgargcraaa gctcgccgtg cg 822

<210> 4

<211> 822

<212> DNA

<213> Artificial Sequence

<220>

<223> Description of Artificial Sequence: Consensus sequence of vibrio

cholera -rpoD

<400> 4

acacgtgaag gtgaaatcga tattgccaag cgcattgaag atggtattaa ccaagttcaa 60

agtgcgattg ctgagtatcc tggaaccatc ccttatattc ttgagcagtt tgatcgtgtt 120

caggccgaag agctacgtct cactgacctg atttcaggtt tcgttgaycc taacgacatg 180

gaaaccgaag cgccaaccgc gactcacatc ggttctgagc tttctgaagc ggatctcgcg 240

gatgaagatg atgctgtcgt cgaagatgaa gacgaagatg aagacgaaga tggcgacggt 300

gaaagcagcg acagcgaaga agaagtcggt atcgaccctg aactggctcg tgagaaattc 360

aatgaactgc gcggyaagtt ccaaaacctg caattagcgg ttaatgaatt tggtcgtgac 420

agtcatcaag cttctgaagc gtcagactta gtgytggata tcttccgtga attccgycta 480

acaccaaagc aattcgacca cttggttgaa actctgcgca cttcaatgga tcgtgttcgc 540

acccaagaac gtttggtrat gaaagcggta gttgaagtcg cgaagatgcc gaagaaatcg 600
ttcatcgccc tatttacagg caatgaatcg aatgaagagt ggctggataa agtccttgct 660
tctgacaagc cttacgtagc gaaagtccgt gagcaagaag aagagatccg ccgttcaatt 720
cagaaactac aaatgatcga gcaagagaca tcactgtctg ttgaacgcat caaagacatc 780
agccgtcgta tgtcaatcgg tgaggcraaa gctcgccgtg cg 822

<210> 5
<211> 885
<212> DNA
<213> Artificial Sequence

<220>
<223> Description of Artificial Sequence: Consensus sequence of vibrio
mimicus -gyrB

<400> 5
gtctccggtg gtctacacgg ggtaggtgtg tcggtagtga atgccctgtc agaaaaagtg 60
ctgctbacca tttatcgtgg tggcaagatt cacacccaaa cttaccatca cggtgtgcca 120
caagcaccgt tgtctgtrgt gggtgagact gagcgtaccg gtactaccgt acgtttctgg 180
cctagtgcac agacttttac caatatcgaa ttccattacg acattctggc taaacgyctg 240
cgtgagctgt cattcctgaa ctctggcgtg tcgatcaagc tgacggatga gcgtgaagaa 300
gataagaaag accacttyat gtatgaaggt ggtattcaag cgtttgtkac ccacttgaac 360
cgtaayaaaa cgccgatcca tgaaaaagta ttccacttca accaagagcg tgaagatggc 420
atcagcgtgg aagtggcaat gcagtggaac gatggtttcc aagaaaacat ctactgcttt 480
accaacaaca tyccacagcg tgatggcggt acccacttag cyggtttccg tggtgcrttg 540
acccgtactt tgaacaacta catggacaaa gaaggcttct cgaagaaagc scaagcrgca 600
acctcgggtg atgatgcgcg tgaaggctta acrgcrgtkg tktcggtgaa agtrccrgat 660
cctaaattct cragccaaac caaagataag ctrgtttctt cggargtgaa atccgcggtt 720
gagtcagcca tgaatgagaa gctggcggat ttcctggcgg aaaacccaag cgaagcgaaa 780

aacgtttgtt cgaagattat tgatgcrgcr cghgctcgtg aagcvgcgcg taaagcacgt 840

gaaatgacyc gtcgtaaagg cgcgctagay ytmgctggtt tgccw 885

<210> 6

<211> 822

<212> DNA

<213> Artificial Sequence

<220>

<223> Description of Artificial Sequence: consensus sequence of vibrio mimicus -rpoD

<400> 6

acacgtgaag gcgaaatcga tattgccaag cgcattgaag atggtattaa ccaagttcaa 60

agtgcgattg ctgagtatcc tggaaccatc ccatacattc ttgaacagtt tgacaaggtt 120

caggcagaag aactacgtct gactgayctg atttctggtt tcgttgatcc taacgacatg 180

gaaaccgaag cgccaactgc tactcacatc ggttcagarc tctctgaagc cgatctcgct 240

gatgaagatg acgaggtcgc ggaggatgaa gacgaggatg aagatgaaga cggcgacggt 300

gaaagyagcg acagcgaaga agaagtgggt attgaccctg agctcgctcg tgagaaattc 360

aatgaactgc gcggcaagtt ccaaaacctg caattagcgg ttaatgaatt tggtcgtgac 420

agtaaccaag catctgaagc ttcaagcctg gtactggata tyttccgcga attccgccta 480

acaccaaaac aatttgacca tttggttgaa actctgcgta cctcgatgga tcgtgttcgt 540

acccaagagc gyttggtgat gaaagcvgtg gttgaagtcg cgaaaatgcc aaagaaatca 600

tttattgcyc trtttacagg caatgaatcg aatgargaat ggctygataa agtrctcgct 660

tctgataarc cttatgtaca aaaagtacgt gagcaagaag acgatattcg ccgctcaatc 720

caaaaactkc agatgatcga acargagact tcactgtctg ttgagcgtat caaagacatc 780

agccgtcgta tgtctatcgg tgaagcgaaa gctcgccgtg cg 822

## Description

### TECHNICAL FIELD

The present invention relates to a method for detecting, identifying, and counting *Vibrio cholerae and Vibrio mimicus* in food inspection, epidemiological environmental inspection, and clinical examination.

### BACKGROUND ART

*Vibrio cholerae* produces a cholera toxin after oral infection and induces violent diarrhea and vomiting. In some cases, the infectious disease bacterium may cause affected patients to die from extreme dehydration. It has been thought that *Vibrio cholerae,* being toxic to humans, is only O1 cholerae that agglutinates by the use of an anti-O1 antibody. It has also been thought that *Vibrio* other than the bacterium is classified as NAG (O1-non-agglutinable)-Vibrio and is not toxic to humans. However, in the Bengal district in India in 1995, a new type of *Vibrio cholerae* having no O1 antigen that causes symptoms similar to those caused by conventional cholerae was isolated, revealing that the new *Vibrio cholerae* strain has a new O-antigen referred to as 0139. This strain is referred to as the Bengal strain and has been revealed to have a cholerae toxin-producing gene similar to that of conventional O1 cholerae, which produces a toxin and induces cholera. Accordingly, it has been shown that *Vibrio* other than O1 cholerae can infect humans and cause cholera. Actually, *Vibrio cholerae* strains having cholera toxin genes are present, in addition to O1 and O139 strains. Such strains induce cholera in humans. However, symptoms due to *Vibrio cholerae* strains other than the O1 and 0139 strains are not treated as infectious diseases administratively.

In the meantime, a non-saccharolytic strain has been rarely detected among non-O1 strains of *Vibrio cholerae.* Davis et al., have examined DNA homology of the strain with *Vibrio cholerae,* thereby showing that the strain is somewhat different from *Vibrio cholerae* and designating the strain as *Vibrio mimicus.* It has been reported that *Vibrio mimicus* is a strain very closely related to *Vibrio cholerae.* Specifically, except saccharolytic ability *(Vibrio cholerae* is positive and *Vibrio mimicus* is negative), the biochemical properties of the two species are very similar (J. Clin. Microbiol. 14, 631-639, 1981) (non-patent document 1). It is known that among *Vibrio mimicus* there are strains having a cholera toxin gene similar to that of *Vibrio cholerae* (Microbiol Immunol 1998, 42, 823-828) (non-patent document 2). *Vibrio mimicus* may thus induce symptoms similar to those of cholera.

Furthermore, among *Vibrio cholerae* having no cholera toxin genes, strains that have been reported to be present are: strains having a thermostable direct hemolysin (tdh) gene (Appl Environ Microbiol 52, 1218-20, 1986) (non-patent document 3) that is a pathogenic factor of *Vibrio parahaemolyticus;* and a strain producing a thermostable enterotoxin of *Escherichia coli* (J. Clin. Invest. 85: 697-705, 1990) (non-patent document 4). They can cause diarrhea and the like. Similarly, among *Vibrio mimicus,* a strain having a tdh gene has been reported to be present (FEMS Microbiol 59: 319-23, 1990) (non-patent document 5), which can cause diarrhea and the like even when it does not produce any cholera toxin.

As described above, a case in which *Vibrio cholerae* having serotype of O1 or O139 (Bengal) and producing a cholera toxin is detected is a subject to measures specified in the "Law Concerning Prevention of Infection of Infectious Diseases and Patients with Infectious Diseases." Moreover, there are also cholera toxin-producing strains among *Vibrio cholerae* other than O1 cholerae and Bengal cholerae, which induce symptoms similar to those of cholera. Furthermore, strains producing no cholera toxins do not cause cholera. However, this does not mean that they are non-pathogenic. Such strains induce acute gastroenteritis, diarrhea, and the like by producing toxins other than the cholera toxin. Therefore, it is required to rapidly and precisely detect the *Vibrio cholerae* bacterial group.

Furthermore, in the case of *Vibrio mimicus,* there are strains producing a cholera toxin that can cause cholera and strains causing acute gastroenteritis, diarrhea, and the like through the production of toxins other than the cholera toxin. Therefore, it is also required to rapidly and precisely detect the *Vibrio mimicus* bacterial group.

Conventional methods of examination using biochemical techniques require skilled technology and much effort and time. To compensate for such deficiencies, precise, rapid, and convenient methods of examination using genes have been attempted for detecting or identifying *Vibrio cholerae* and *Vibrio mimicus.*

Primers for detecting the gene encoding the cholera toxin that causes cholera are already known (J. Biol. Chem. 1983, 258, 13722-13726) (non-patent document 6). However, it is impossible to detect *Vibrio cholerae* and *Vibrio mimicus,* which do not have any cholera toxin gene but which produce other toxins through the use of such primers.

In the meantime, when *Vibrio cholerae* and *Vibrio mimicus* are compared in terms of the full-length nucleotide sequence of a 16S rRNA gene, only 6 among 1,456 nucleotides are found to differ (Int. J. Syst. Bacteriol. 44, 416-426, 1994) (non-patent document 7). Thus, it is impossible to clearly distinguish between the two. Accordingly, there is a report wherein *Vibrio cholerae* and *Vibrio mimicus* were compared in terms of the 16S-23S rRNA intergenic spacer region so as to show that they can be classified and primers with which only *Vibrio cholerae* can be detected were produced (Appl. Environ. Microbiol. 65, 2202-2208, 1999) (non-patent document 8). However, there is also a report wherein *Vibrio mimicus* was detected by mistake when the primers were used (Appl. Environ. Microbiol. 67, 2360-2364 2001) (non-patent document 9).

### SUMMARY OF THE INVENTION

As described above, it has been impossible to detect with high accuracy *Vibrio cholerae* and *Vibrio mimicus* by the existing genetic detection methods.

What is common among conventional genetic screening methods is that they ignore the fact that a bacterial "species" is a population containing genetic diversity. The use of a nucleotide sequence of a single bacterial strain that is inferred to be a member of a bacterial population as a common sequence for such population or a sequence representing such population is very dangerous in terms of molecular evolutional characteristics of genes, which rapidly accumulate neutral mutations. Specifically, there is concern that such use could cause misidentification such that a bacterial strain to be detected could not be detected because of inhibited amplification due to a slight mutation in a primer region. There is also concern that a closely related strain not to be detected would be detected because of insufficient primer specificity. Hence, it has been required to produce a high-performance and specific gene amplification primer for detecting, identifying, or quantifying *Vibrio cholerae* and *Vibrio mimicus* and a gene amplification primer for specifically detecting, identifying, or quantifying *Vibrio cholerae* and *Vibrio mimicus,* which have proven backgrounds of specificity, low possibility of misidentification, and practically sufficient amplification efficiency and amplification specificity.

To produce a method for specifically detecting a gene of a bacterial phylogenetic group, there is a need to collect as many nucleotide sequences as possible from a biological group to be detected and from biological groups phylogenetically close to these groups. Moreover, a gene targeted for specific detection should have a sufficiently unique nucleotide sequence so that it is distinguishable from the most closely related organisms. In order to satisfy these conditions, a target gene must have a sufficiently rapid rate of evolution. In addition, as in the case of a gene that is transmitted horizontally at a high frequency (e.g., a toxin gene of *Vibrio parahaemolyticus),* a gene that exists independently of the phylogenetic line cannot be used. Proteins encoded by a *gyrB* gene and an *rpoD* gene used as targets in the present invention are essential for survival. Because of this reason, these genes are hardly transmitted horizontally and have appropriate rates of evolution, so that they are preferably used for bacterial phylogenetic analysis (Int. J. Syst. Bacteriol. 1998, 48, 813-819; Int. J. Syst. Bacteriol. 1999, 49, 87-95). We have already developed a convenient method for determining a nucleotide sequence that involves subjecting the *gyrB* gene and the *rpoD* gene to the PCR direct sequencing method (JP Patent Publication (Kokai) Nos. 07-213229 A (1995) and 08-256798 A (1996)). Furthermore, we have already isolated large quantities of *Vibrio cholerae.* In the meantime, regarding a known stock strain of *Vibrio mimicus* and those reported to be closely related to this bacterium according to analysis based on a 16S rRNA sequence, *Listonella anguillarum, V ordalii, V. diazotrophicus, V. vulnificus, V. navarrensis, V. metschnikovii,* and *V cincinnatiensis* (International Journal of Systematic and Evolutionary Microbiology 51, 1449-1456 (2001)), we have analyzed the partial nucleotide sequences of the *gyrB* and *rpoD* genes of bacterial strains listed in Table 1 and conducted molecular phylogenetic analysis based on such sequences, thereby revealing the phylogenetic relationship.

**Table 1.**

| Strains used | | | |
|---|---|---|---|
| *V. cholerae* | 29 strains | Clinically isolated | 29 strains |
| | | strains | |
| *V. mimicus* | 4 strains | ATCC 33653 T | |
| | | ATCC 33654 | |
| | | ATCC 33655 | |
| | | ATCC 700326 | |
| Other stock strains | 52 strains | | |
| of the genus *Vibrio* | | | |
| *V. vulnificus* | 32 strains | ATCC 27562 T | |
| | | ATCC 29306 | |
| | | ATCC 29307 | |
| | | ATCC 33147 | |
| | | ATCC 33148 | |
| | | ATCC 33149 | |
| | | ATCC 33814 | |
| | | ATCC 33815 | |
| | | ATCC 33816 | |
| | | ATCC 33817 | |
| | | ATCC 43382 | |
| | | ATCC BAA-86 | |
| | | ATCC BAA-87 | |
| | | ATCC BAA-88 | |
| | | ATCC BAA-89 | |
| | | ATCC BAA-90 | |
| | | JCM 3726 | |
| | | JCM 3727 | |
| | | JCM 3728 | |
| | | JCM 3729 | |
| | | JCM 3730 | |
| | | JCM 3731 | |
| | | Strains isolated | 10 strains |
| | | from environment | |
| *V. diazotrophicus* | | ATCC 33466 T | |
| *V. navarrensis* | | ATCC 51183 T | |
| *V. metschnikovii* | | ATCC 700040 T | |
| *V. cincinnatiensis* | | ATCC 35912 T | |
| *V. ordalii* | | NCIMB 2167 T | |
| *Listonella* | | NCIMB 6 T | |
| *anguillarum* | | | |
| *V. hollisae* | | ATCC 33564 T | |
| *V. alginolyticus* | | IFO 15630 T | |
| *V. campbellii* | | IFO 15631 T | |
| *V. carchariae* | | IFO 15632 T | |
| *V. harveyi* | | IFO 15634 T | |
| *V. nereis* | | IFO 15637 T | |
| V. | | | |
| *parahaemolyticus* | | IFO 12711 T | |
| *V. proteolyticus* | | IFO 13287 T | |
| *V. tubiashii* | | IFO 15644 T | |
| *Vibrio.* sp | 5 strains | Strains isolated | |
| | | from food | |
| | | | Total 85 strains |

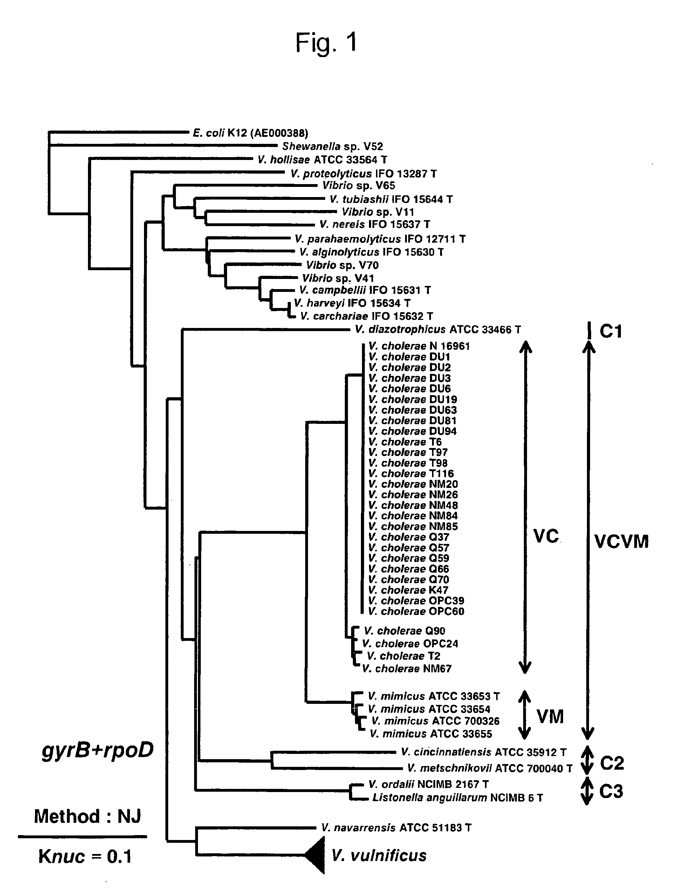

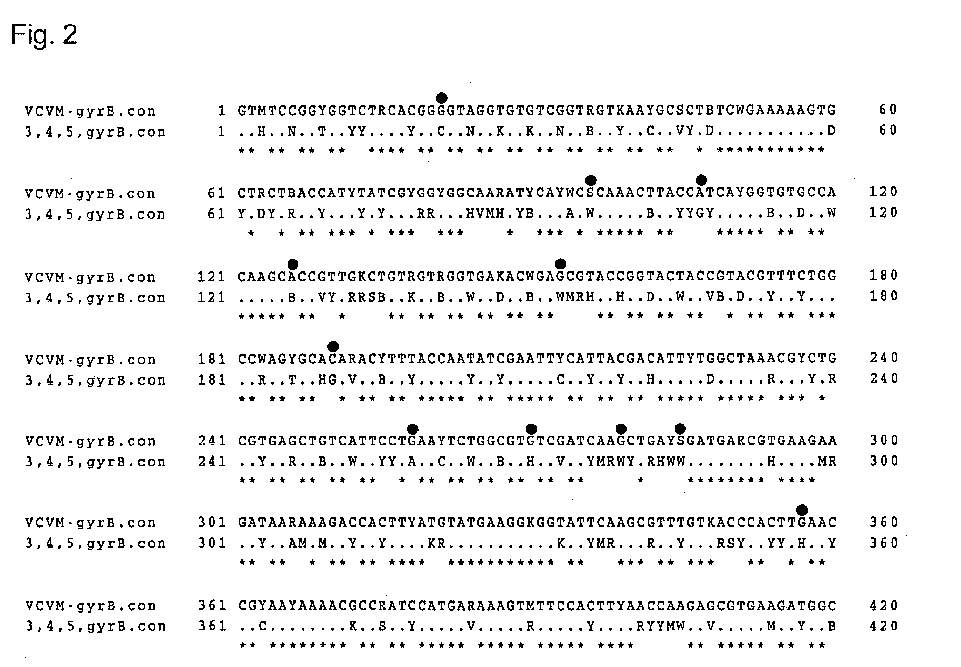

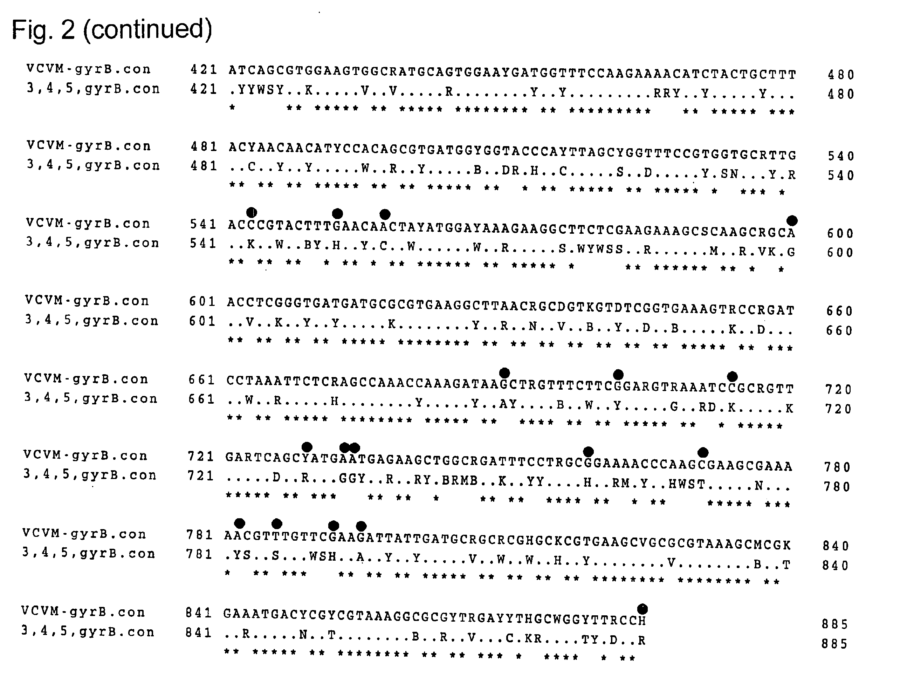

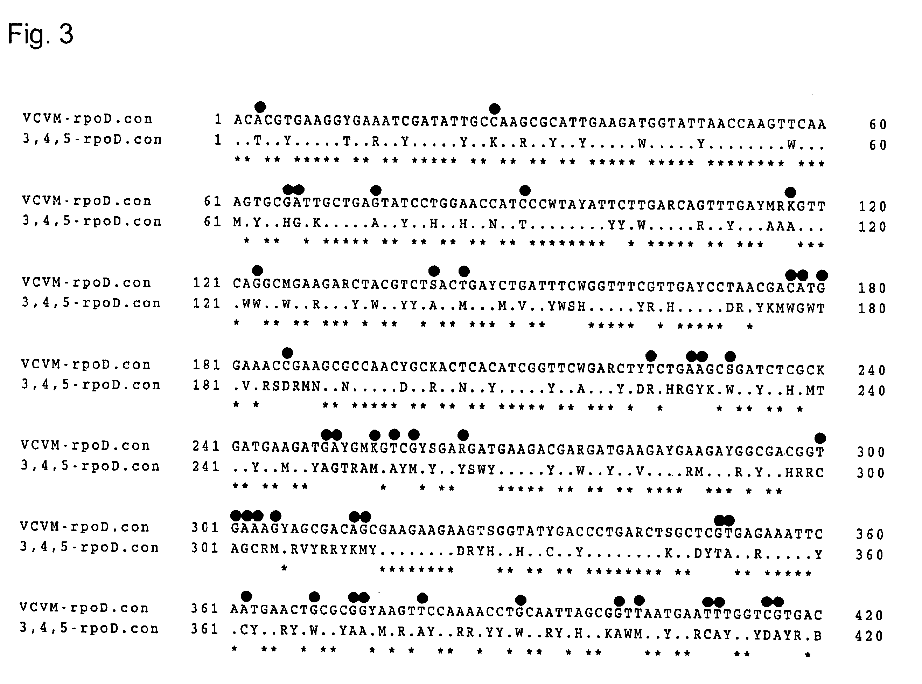

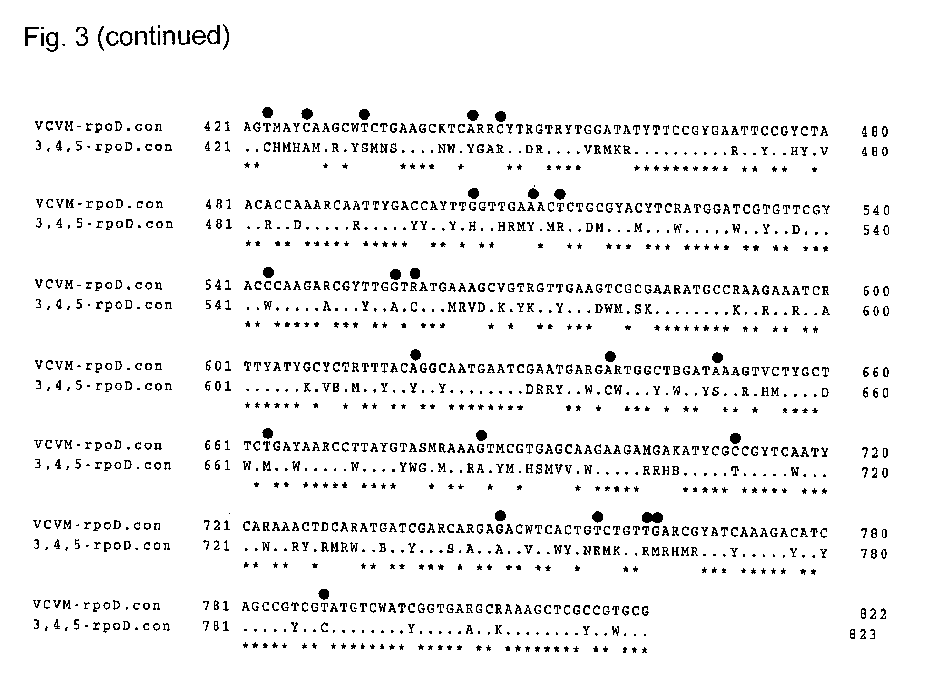

Specifically, after the test strains had been subjected to enrichment culture using brain heart infusion media supplemented with 2% NaCl, chromosomal DNA was extracted using a PUREGENE DNA Isolation Kit (Gentra SYSTEMS). Using the extracted DNA as a template, a *gyrB* gene fragment with a length of approximately 900 bp (a region corresponding to positions 331 to 1212 of the nucleotide sequence or positions 111 to 404 of the amino acid sequence of *Escherichia coli* strain K-12) was amplified by PCR using *gyrB* universal amplification primers UP-1E and AprU. Furthermore, similarly, an *rpoD* gene fragment with a length of approximately 800 bp (a region corresponding to positions 334 to 1125 of the nucleotide sequence and positions 112 to 375 of the amino acid sequence of *Escherichia coli* strain K-12) was amplified by PCR using *rpoD* universal amplification primers 70F-M13 and 70R-M13. The obtained PCR product was subjected to 1% agarose gel electrophoresis, and then stained with ethidium bromide. Amplified products were confirmed to be present under UV irradiation, and then purified using a Wizard PCR Preps DNA Purification System (Promega), thereby preparing templates for sequence reaction. Cycle sequence reaction was conducted using as primers M13R and M13-21 sequences previously added to the universal primers and an ABI PRISM BigDye Terminator Cycle Sequencing Ready Reaction Kit (PE Applied Biosystems). The nucleotide sequences were analyzed using an ABI PRISM 310 Genetic Analyzer (PE Applied Biosystems). Upon molecular phylogenetic analysis using the determined nucleotide sequences, to more precisely understand closely related species of *Vibrio cholera* and *mimicus,* analysis was conducted after linking partial sequences of the *gyrB* and *rpoD* genes. The obtained nucleotide sequences were subjected to multiple alignment analysis using a Clustal W computer program. Subsequently, through the use of a PHYLIP computer program package, a molecular phylogenetic tree was produced by the neighbor-joining method based on genetic distance calculated using Kimura's 2-parameter model. As a result, it was shown that *Vibrio cholerae* and *mimicus* belong to mono phyletic line different from those of other bacteria of the genus *Vibrio.* Specifically, it was suggested that *Vibrio cholerae* and *mimicus* each forms an independent mono phyletic group (Fig. 1). Hence, to establish a genetic screening method that can detect only *Vibrio cholerae* and *mimicus* bacterial groups, firstly differences in nucleotide sequences among closely related species were revealed. That is, nucleotide positions that are conserved within the *Vibrio cholerae* and *mimicus* groups but differ from other bacteria of the genus *Vibrio* were identified. Specifically, consensus sequences of the phylogenetic groups to which *Vibrio cholerae* and *mimicus* belong were found and compared with consensus sequences of C1 to C3 clusters in Fig. 1 determined to be closely related to this phylogenetic line based on the results of molecular phylogenetic analysis. Thus, a phylogenetically specific information map was produced (Figs. 2 and 3). The following positions are specific to the phylogenetic line to which *Vibrio cholerae* and *mimicus* belong: as shown in Fig. 2, in the case of the *gyrB* gene, positions (hereinafter also referred to as nucleotide number) 21, 96, 107, 126, 153, 190, 258, 270, 279, 285, 357, 543, 552, 557, 600, 690, 702, 714, 729, 733, 734, 759, 771, 782, 786, 792, 795, and 885 of SEQ ID NO: 1 in the sequence listing; and as shown in Fig. 3, in the case of the *rpoD* gene, positions 3, 27, 66, 67, 75, 90, 117, 123, 141, 144, 177, 178, 180, 186, 223, 227, 228, 231, 250, 251, 255, 257, 259, 264, 300, 301, 302, 303, 305, 313, 314, 350, 351, 362, 369, 373, 374, 380, 390, 400, 402, 409, 410, 415, 416, 423, 427, 433, 444, 447, 504, 510, 513, 543, 556, 558, 618, 638, 649, 663, 685, 711, 747, 757, 762, 763, and 789 of SEQ ID NO: 2 in the sequence listing. The use of sequences specific to this phyletic line containing these characteristic nucleotides enables the design of probes having high specificity and gene amplification primers having high specificity and excellent amplification efficiency. For example, it becomes possible to design such a primer containing 15 or more continuous nucleotides (containing nucleotides that differ from those of closely related species so as to always contain positions that differ in terms of nucleotides from those of closely related species) of nucleotide sequences of the *gyrB* and *rpoD* genes, preferably containing 20 or more nucleotides, and further preferably containing 20 or more nucleotides and 40 or fewer continuous nucleotides of nucleotide sequences of the *gyrB* and *rpoD* genes. Similarly, it also becomes possible to design a probe containing 15 or more continuous nucleotides (containing nucleotides that differ from those of closely related species so as to always contain positions that differ in terms of nucleotides from those of closely related species) of nucleotide sequences of the *gyrB* and *rpoD* genes, preferably containing 20 or more nucleotides, and further preferably containing 20 or more nucleotides and 100 or fewer continuous nucleotides of nucleotide sequences of the *gyrB* and *rpoD* genes. Furthermore, to produce the primers and the probes, a region containing the above different nucleotides at high frequencies (containing 2 or more such nucleotides) can be preferably used, such as: in the case of the *gyrB* gene, a region containing positions 96 and 107, a region containing 2 or more positions of any of positions 258, 270, 279, or 285, a region containing 2 or more positions of any of positions 543, 552, or 557, a region containing 2 or more positions of any of positions 690, 702, or 714, and a region containing 2 or more positions of any of positions 729, 733, or 734, a region containing 759 and 771, and a region containing 2 or more positions of any of positions 782, 786, 792, or 795; and in the case of the *rpoD* gene, a region containing 2 or more positions of any of positions 66, 67, 75, or 90, a region containing 2 or more positions of any of positions 177, 178, 180, or 186, a region containing 2 or more positions of any of positions 223, 227, 228, or 231, a region containing 2 or more positions of any of positions 250, 251, 255, 257, 259, or 264, a region containing 2 or more positions of any of positions 300, 301, 302, 303, 305, 313, or 314, a region containing 2 or more positions of any of positions 362, 369, 373, 374, or 380, a region containing 2 or more positions of any of positions 400, 402, 409, 410, 415, or 416, a region containing 2 or more positions of any of positions 423, 427, 433, 444, or 447, a region containing 2 or more positions of any of positions 504, 510, or 513, a region containing 2 or more positions of any of positions 543, 556, or 558, and a region containing 2 or more positions of any of positions 747, 757, 762, or 763. Furthermore, in the case of primers, the 3'-terminus is preferably a nucleotide specific to *Vibrio cholerae* and *mimicus* bacterial groups. The present invention encompasses a kit for detecting, quantifying, or identifying *Vibrio cholera* and *mimicus* using these primers and probes in combination with other reagents.

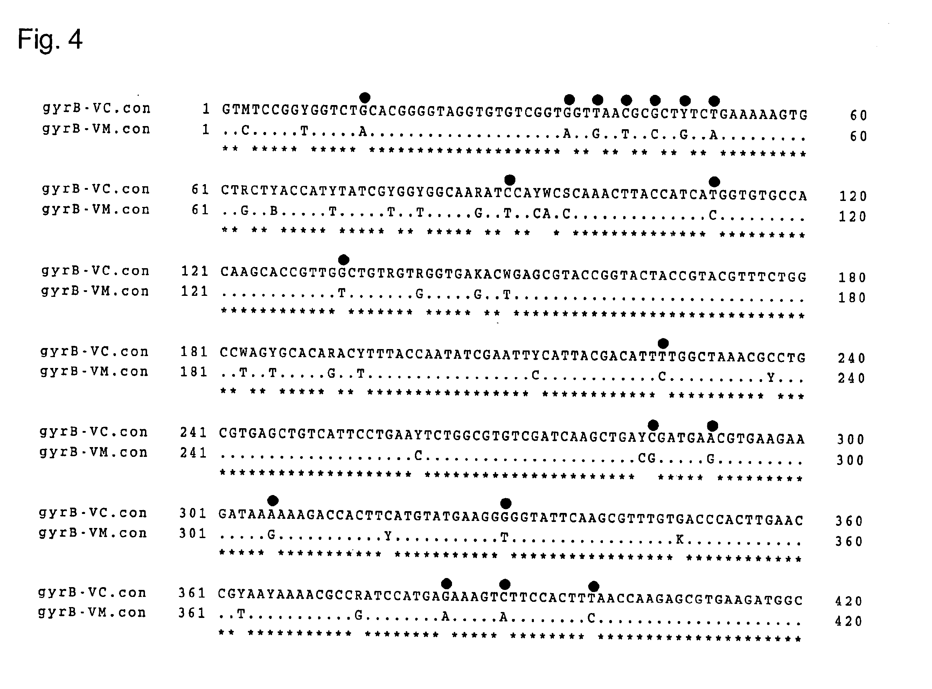

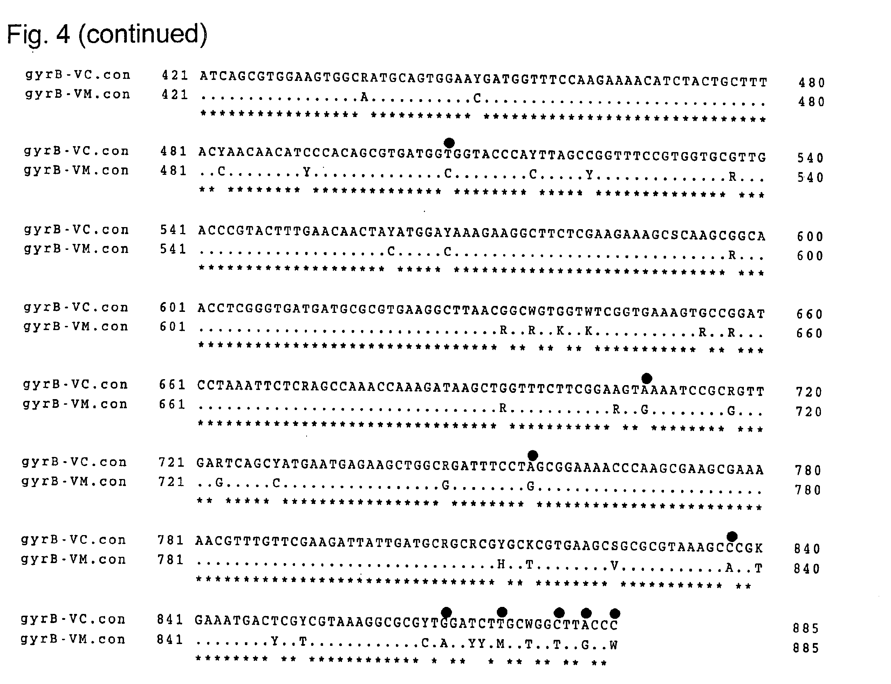

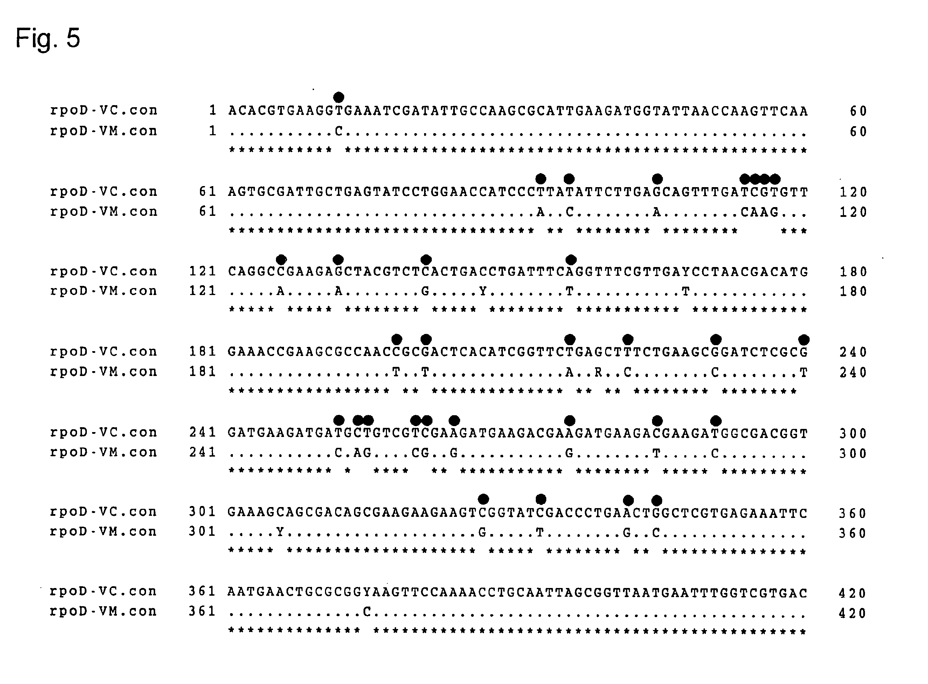

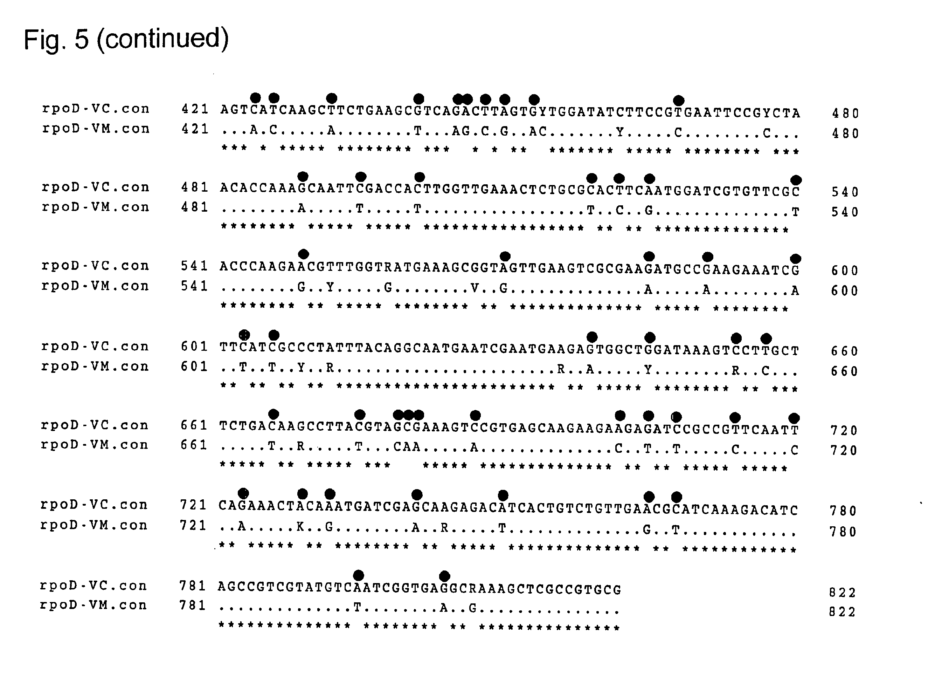

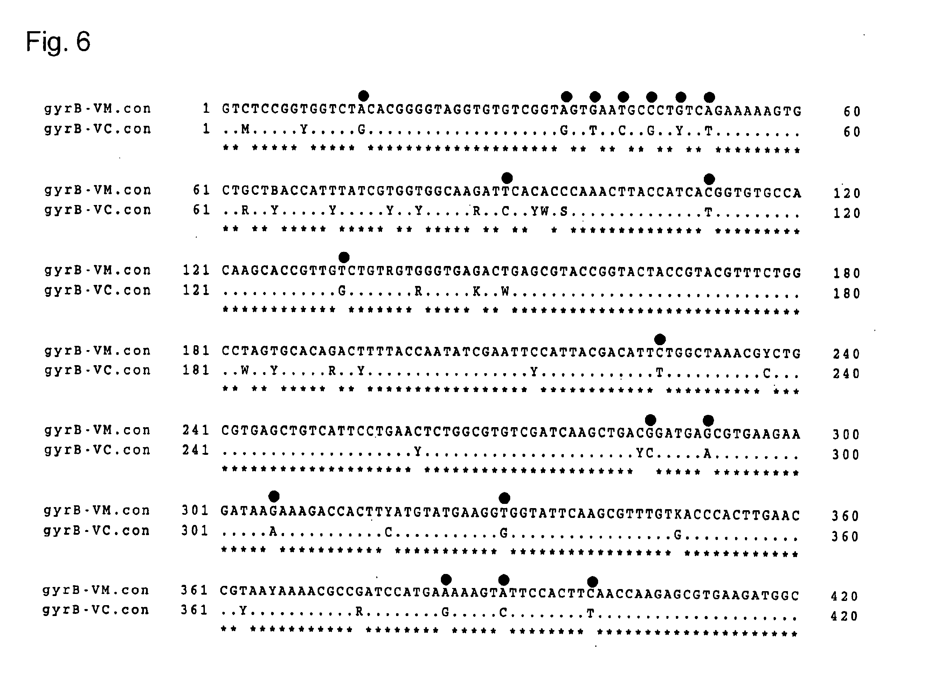

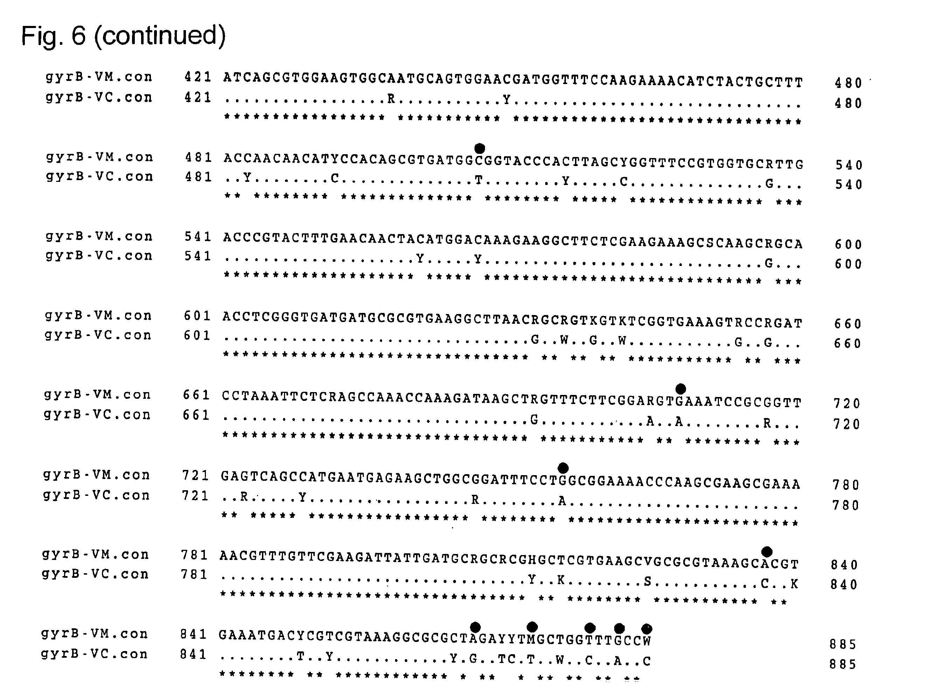

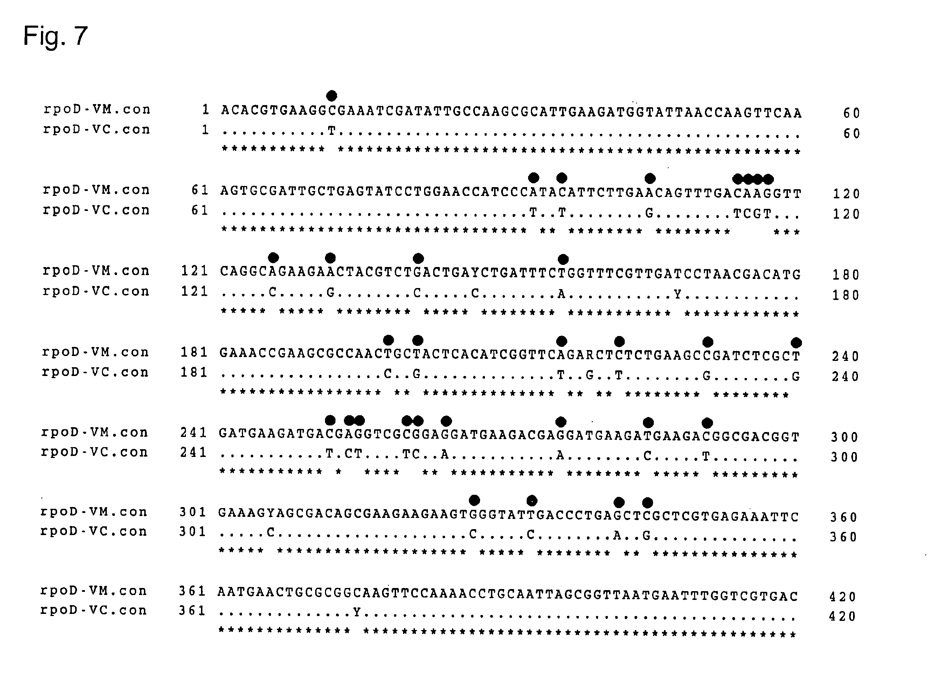

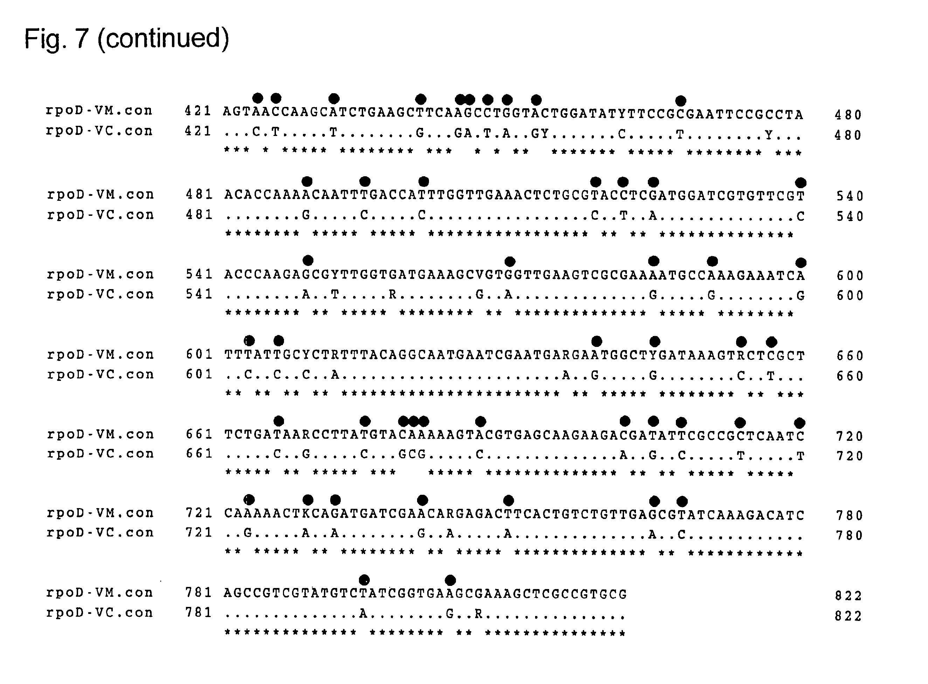

In the meantime, *Vibrio cholerae* and *mimicus* bacterial groups, that is, the *Vibrio cholerae* bacterial group and the *Vibrio mimicus* bacterial group, further form an independent mono phyletic group (Fig. 1). Hence, to establish a genetic screening method that can separately detect the *Vibrio cholerae* and *mimicus* bacterial groups, regions that are conserved within each of the *Vibrio cholerae* and *mimicus* groups, and nucleotide positions that differ from each other within the same were identified in a manner similar to the above. Specifically, consensus sequences of phylogenetic groups to which *Vibrio cholerae* and *mimicus* belong were compared with each other, so that a phylogenetically-specific information map was produced (Figs. 4, 5, 6, and 7). Regarding the mono phyletic group to which *Vibrio cholerae* belongs, the following positions are specific: as shown in Fig. 4, in the case of the *gyrB* gene, positions 15, 36, 39, 42, 45, 48, 51, 90, 111, 133, 226, 285, 291, 306, 330, 384, 390, 399, 507, 708, 756, 837, 867, 873, 879, 882, and 885 of SEQ ID NO: 3 in the sequence listing; and as shown in Fig. 5, in the case of the *rpoD* gene, positions 12, 93, 96, 105, 114, 115, 116, 117, 126, 132, 141, 156, 198, 201, 216, 222, 231, 240, 252, 254, 255, 260, 261, 264, 276, 285, 291, 327, 333, 342, 345, 424, 426, 432, 441, 445, 446, 448, 450, 453, 468, 489, 495, 501, 519, 522, 525, 540, 549, 570, 585, 591, 600, 603, 606, 639, 645, 654, 657, 666, 675, 679, 680, 681, 687, 702, 705, 708, 714, 720, 723, 729, 732, 741, 750, 765, 768, 795, and 804 of SEQ ID NO: 4 in the sequence listing. Regarding the phyletic group to which *Vibrio mimicus* belongs, the following positions are specific: as shown in Fig. 6, in the case of the *gyrB* gene, positions 15, 36, 39, 42, 45, 48, 51, 90, 111, 133, 226, 285, 291, 306, 330, 384, 390, 399, 507, 708, 756, 837, 867, 873, 879, 882, and 885 of SEQ ID NO: 5 in the sequence listing; and as shown in Fig. 7, in the case of the *rpoD* gene, positions 12, 93, 96, 105, 114, 115, 116, 117, 126, 132, 141, 156, 198, 201, 216, 222, 231, 240, 252, 254, 255, 260, 261, 264, 276, 285, 291, 327, 333, 342, 345, 424, 426, 432, 441, 445, 446, 448, 450, 453, 468, 489, 495, 501, 519, 522, 525, 540, 549, 570, 585, 591, 600, 603, 606, 639, 645, 654, 657, 666, 675, 679, 680, 681, 687, 702, 705, 708, 714, 720, 723, 729, 732, 741, 750, 765, 768, 795, and 804 of SEQ ID NO: 6 in the sequence listing. The use of sequences that are specific to the phyletic group to which *Vibrio cholera* or *Vibrio mimicus* belongs and containing these characteristic nucleotides enables the design of probes having high specificity and gene amplification primers having high specificity and excellent amplification efficiency. For example, it becomes possible to design a primer containing 15 or more continuous nucleotides (containing nucleotides that differ between the two bacterial species so as to always contain nucleotide positions that are different between *Vibrio cholerae* and *Vibrio mimicus)* of nucleotide sequences of the *gyr B* and *rpoD* genes, preferably containing 20 or more nucleotides, and further preferably containing 20 or more nucleotides and 40 or fewer continuous nucleotides of nucleotide sequences of the *gyrB* and *rpoD* genes. Similarly, it also becomes possible to design a probe containing 15 or more continuous nucleotides (containing nucleotides that differ between the 2 bacterial species so as to always contain nucleotide positions that differ between *Vibrio mimicus* and *Vibrio cholerae)* of nucleotide sequences of the *gyrB* and *rpoD* genes, preferably containing 20 or more nucleotides, and further preferably containing 20 or more nucleotides and 100 or fewer continuous nucleotides of nucleotide sequences of the *gyrB* and *rpoD* genes. Furthermore, to produce the primers and the probes, a region containing the above different nucleotides at high frequencies (containing 2 or more such nucleotides) can be preferably used, such as: in the case of the *gyrB* gene, a region containing 2 or more positions of any of positions 36, 39, 42, 45, 48, or 51, a region containing 2 or more positions of any of positions 285, 291, or 306, a region containing 2 or more positions of any of positions 384, 390, or 399, and a region containing 2 or more positions of any of positions 867, 873, 879, 882, or 885; and in the case of the *rpoD* gene, a region containing 2 or more positions of any of positions 93, 96, 105, 114, 115, 116, or 117, a region containing 2 or more positions of any of positions 126, 132, or 141, a region containing 2 or more positions of any of positions 216, 222, 231, or 240, a region containing 2 or more positions of any of positions 252, 254, 255, 260, 261, or 264, a region containing 2 or more positions of any of positions 276, 285, or 291, a region containing 2 or more positions of any of positions 327, 333, 342, or 345, a region containing 2 or more positions of any of positions 424, 426, 432, 441, 445, or 446, a region containing 2 or more positions of any of positions 448, 450, 453, or 468, a region containing 2 or more positions of any of positions 489, 495, or 501, a region containing 2 or more positions of any of positions 519, 522, 525, or 540, a region containing 2 or more positions of any of positions 585, 591, 600, 603, or 606, a region containing 2 or more positions of any of positions 639, 645, 654, or 657, a region containing 2 or more positions of any of positions 666, 675, 679, 680, 681, or 687, a region containing 2 or more positions of any of positions 702, 705, 708, 714, 720, or 723, and a region containing 2 or more positions of any of positions 729, 732, 741, or 750. Furthermore, in the case of primers, the 3'-terminus is preferably a nucleotide specific to *Vibrio cholerae* or *mimicus.*

Specifically, primers for detecting, quantifying, or identifying the *Vibrio cholerae* and *mimicus* bacterial groups are as follows.
(1) In the case of the *gyrB* gene, examples of such gene amplification primers include: a gene amplification primer containing a strand or a complementary strand thereof of 15 or more continuous nucleotides of SEQ ID NO: 1 containing nucleotides at positions 96 and 107 of SEQ ID NO: 1; a gene amplification primer comprising a strand or a complementary strand thereof of 15 or more continuous nucleotides of SEQ ID NO: 1 containing 2 or more nucleotides at any of positions 258, 270, 279, or 285 of SEQ ID NO: 1; a gene amplification primer comprising a strand or a complementary strand thereof of 15 or more continuous nucleotides of SEQ ID NO: 1 containing 2 or more nucleotides at any of positions 543, 552, or 557 of SEQ ID NO: 1; a gene amplification primer comprising a strand or a complementary strand thereof of 15 or more continuous nucleotides of SEQ ID NO: 1 containing 2 or more nucleotides at any of positions 690, 702, or 714 of SEQ ID NO: 1; a gene amplification primer containing a strand or a complementary strand thereof of 15 or more continuous nucleotides of SEQ ID NO: 1 containing 2 or more nucleotides at any of positions 729, 733, or 734 of SEQ ID NO: 1; a gene amplification primer containing a strand or a complementary strand thereof of 15 or more continuous nucleotides of SEQ ID NO: 1 containing nucleotides at positions 759 and 771 of SEQ ID NO: 1; and a gene amplification primer containing a strand or a complementary strand thereof of 15 or more continuous nucleotides of SEQ ID NO: 1 containing 2 or more nucleotides at any of positions 782, 786, 792, or 795 of SEQ ID NO: 1. More specific examples include a gene amplification primer that is any one of 5'-tycaywcscaaacttacca-3' or a complementary strand corresponding thereto, 5'-gaaytctggcgtgtcgatcaag-3' or a complementary strand corresponding thereto, 5'-catrtagttgttcaaagtacgg-3' or a complementary strand corresponding thereto, 5'-ggatttyacytccgaagaaacyagc-3' or a complementary strand corresponding thereto, 5'-ygccagcttctcattcatr-3' or a complementary strand corresponding thereto, 5'-cgcttcgcttgggttttcc-3' or a complementary strand corresponding thereto, or 5'-caataatcttcgaacaaacgt-3'or a complementary strand corresponding thereto and gene amplification primers that contain the strands or the complementary strands thereof comprising the above sequences.
(2) In the case of the *rpoD* gene, examples of such gene amplification primers include: a gene amplification primer containing a strand or a complementary strand thereof of 15 or more continuous nucleotides containing 2 or more nucleotides of SEQ ID NO: 2 at any of positions 66, 67, 75 or 90 of SEQ ID NO: 2; a gene amplification primer containing a strand or a complementary strand thereof of 15 or more continuous nucleotides of SEQ ID NO: 2 containing 2 or more nucleotides at any of positions 177, 178, 180, or 186 of SEQ ID NO: 2; a gene amplification primer containing a strand or a complementary strand thereof of 15 or more continuous nucleotides of SEQ ID NO: 2 containing 2 or more nucleotides at any of positions 223, 227, 228, or 231 of SEQ ID NO: 2; a gene amplification primer containing a strand or a complementary strand thereof of 15 or more continuous nucleotides of SEQ ID NO: 2 containing 2 or more nucleotides at any of positions 250, 251, 255, 257, 259, or 264 of SEQ ID NO: 2; a gene amplification primer containing a strand or a complementary strand thereof of 15 or more continuous nucleotides of SEQ ID NO: 2 containing 2 or more nucleotides at any of positions 300, 301, 302, 303, 305, 313, or 314 of SEQ ID NO: 2; a gene amplification primer containing a strand or a complementary strand thereof of 15 or more continuous nucleotides of SEQ ID NO: 2 containing 2 or more nucleotides at any of positions 362, 369, 373, 374, or 380 of SEQ ID NO: 2; a gene amplification primer containing a strand or a complementary strand thereof of 15 or more continuous nucleotides of SEQ ID NO: 2 containing 2 or more nucleotides at any of positions 400, 402, 409, 410, 415, or 416 of SEQ ID NO: 2; a gene amplification primer containing a strand or a complementary strand thereof of 15 or more continuous nucleotides of SEQ ID NO: 2 containing 2 or more nucleotides at any of positions 423, 427, 433, 444, or 447 of SEQ ID NO: 2; a gene amplification primer containing a strand or a complementary strand thereof of 15 or more continuous nucleotides of SEQ ID NO: 2 containing 2 or more nucleotides at any of positions 504, 510, or 513 of SEQ ID NO: 2; a gene amplification primer containing a strand or a complementary strand thereof of 15 or more continuous nucleotides of SEQ ID NO: 2 containing 2 or more nucleotides at any of positions 543, 556, or 558 of SEQ ID NO: 2; and a gene amplification primer containing a strand or a complementary strand thereof of 15 or more continuous nucleotides of SEQ ID NO: 2 containing 2 or more nucleotides at any of positions 747, 757, 762, or 763 of SEQ ID NO: 2. More specific examples include a gene amplification primer that is any one of 5'-gattgctgagtatcctggaaccatc-3' or a complementary strand corresponding thereto, 5'-gaycctaacgacatggaaacc-3' or a complementary strand corresponding thereto, 5'-ttcwgarctytctgaagcs-3' or a complementary strand corresponding thereto, 5'-agatgaygmkgtcgysgar-3' or a complementary strand corresponding thereto, 5'-cgacggtgaaagyagcgacag-3' or a complementary strand corresponding thereto, 5'-caatgaactgcgcggyaagtt-3'or a complementary strand corresponding thereto, 5'-gtcacgaccaaattcattaac-3'or a complementary strand corresponding thereto, 5'-gyytgamgcttcagawgcttgrtka-3' or a complementary strand corresponding thereto, 5'-ygargtrcgcagagtttcaacc-3' or a complementary strand corresponding thereto, 5'-catyaccaarcgytcttgg-3' or a complementary strand corresponding thereto, or 5'-cgytcaacagacagtgawgtc-3' or a complementary strand corresponding thereto and gene amplification primers that contain the strands or the complementary strands thereof comprising the above sequences.

Primers for *Vibrio cholerae* are as follows.
(1) In the case of *gyrB,* examples of such gene amplification primers include: a gene amplification primer containing a strand or a complementary strand thereof of 15 or more continuous nucleotides of SEQ ID NO: 3 containing 2 or more nucleotides at any of positions 36, 39, 42, 45, 48, or 51 of SEQ ID NO: 3; a gene amplification primer containing a strand or a complementary strand thereof of 15 or more continuous nucleotides of SEQ ID NO: 3 containing 2 or more nucleotides at any of positions 285, 291, or 306 of SEQ ID NO: 3; a gene amplification primer containing a strand or a complementary strand thereof of 15 or more continuous nucleotides of SEQ ID NO: 3 containing 2 or more nucleotides at any of positions 384, 390, or 399 of SEQ ID NO: 3; or a gene amplification primer containing a strand or a complementary strand thereof of 15 or more continuous nucleotides of SEQ ID NO: 3 containing 2 or more nucleotides at any of positions 867, 873, 879, 882, or 885 of SEQ ID NO: 3. More specific examples include a gene amplification primer comprising 5'-ggtggttaacgcgctytct-3' or a complementary strand corresponding thereto, a gene amplification primer comprising 5'-ycgatgaacgtgaagaagataaa-3' or a complementary strand corresponding thereto, a gene amplification primer comprising 5'-tgagaaagtcttccacttt-3' or a complementary strand corresponding thereto, a gene amplification primer comprising 5'-gttaaagtggaagactttc-3' or a complementary strand corresponding thereto, and a gene amplification primer comprising 5'-gggtaagccwgcaagatcc-3' or a complementary strand corresponding thereto and gene amplification primers that contain the strands or the complementary strands thereof comprising the above sequences.
(2) In the case of *rpoD,* examples of such gene amplification primers include: a gene amplification primer containing a strand or a complementary strand thereof of 15 or more continuous nucleotides of SEQ ID NO: 4 containing 2 or more nucleotides at any of positions 93, 96, 105, 114, 115, 116, or 117 of SEQ ID NO: 4; a gene amplification primer containing a strand or a complementary strand thereof of 15 or more continuous nucleotides of SEQ ID NO: 4 containing 2 or more nucleotides at any of positions 126, 132, or 141 of SEQ ID NO: 4; a gene amplification primer containing a strand or a complementary strand thereof of 15 or more continuous nucleotides of SEQ ID NO: 4 containing 2 or more nucleotides at any of positions 216, 222, 231, or 240 of SEQ ID NO: 4; a gene amplification primer containing a strand or a complementary strand thereof of 15 or more continuous nucleotides of SEQ ID NO: 4 containing 2 or more nucleotides at any of positions 252, 254, 255, 260, 261, or 264 of SEQ ID NO: 4; a gene amplification primer containing a strand or a complementary strand thereof of 15 or more continuous nucleotides of SEQ ID NO: 4 containing 2 or more nucleotides at any of positions 276, 285, or 291 of SEQ ID NO: 4; a gene amplification primer containing a strand or a complementary strand thereof of 15 or more continuous nucleotides of SEQ ID NO: 4 containing 2 or more nucleotides at any of positions 327, 333, 342, or 345 of SEQ ID NO: 4; a gene amplification primer containing a strand or a complementary strand thereof of 15 or more continuous nucleotides containing 2 or more nucleotides at any of positions 424, 426, 432, 441, 445, or 446 of SEQ ID NO: 4; a gene amplification primer containing a strand or a complementary strand thereof of 15 or more continuous nucleotides of SEQ ID NO: 4 containing 2 or more nucleotides at any of positions 448, 450, 453, or 468 of SEQ ID NO: 4; a gene amplification primer containing a strand or a complementary strand thereof of 15 or more continuous nucleotides of SEQ ID NO: 4 containing 2 or more nucleotides at any of positions 489, 495, or 501 of SEQ ID NO: 4; a gene amplification primer containing a strand or a complementary strand thereof of 15 or more continuous nucleotides of SEQ ID NO: 4 containing 2 or more nucleotides at any of positions 519, 522, 525, or 540 of SEQ ID NO: 4; a gene amplification primer containing a strand or a complementary strand thereof of 15 or more continuous nucleotides of SEQ ID NO: 4 containing 2 or more nucleotides at any of positions 585, 591, 600, 603, or 606 of SEQ ID NO: 4; a gene amplification primer containing a strand or a complementary strand thereof of 15 or more continuous nucleotides of SEQ ID NO: 4 containing 2 or more nucleotides at any of positions 639, 645, 654, or 657 of SEQ ID NO: 4; a gene amplification primer containing a strand or a complementary strand thereof of 15 or more continuous nucleotides of SEQ ID NO: 4 containing 2 or more nucleotides at any of positions 679, 680, 681, 687, or 702 of SEQ ID NO: 4; a gene amplification primer containing a strand or a complementary strand thereof of 15 or more continuous nucleotides of SEQ ID NO: 4 containing 2 or more nucleotides at any of positions 705, 708, 714, 720, or 723 of SEQ ID NO: 4; and a gene amplification primer containing a strand or a complementary strand thereof of 15 or more continuous nucleotides of SEQ ID NO: 4 containing 2 or more nucleotides at any of positions 729, 732, 741, or 750 of SEQ ID NO: 4. More specific examples include a gene amplification primer comprising 5'-attcttgagcagtttgatcgt-3' or a complementary strand corresponding thereto, a gene amplification primer comprising 5'-caggccgaagagctacgtctc-3' or a complementary strand corresponding thereto, a gene amplification primer comprising 5'-tgagctttctgaagcggatctcgcg-3' or a complementary strand corresponding thereto, a gene amplification primer comprising 5'-gaagatgatgctgtcgtcgaa-3' or a complementary strand corresponding thereto, a gene amplification primer comprising 5'-gaagatgaagacgaagat-3' or a complementary strand corresponding thereto, a gene amplification primer comprising 5'-cggtatcgaccctgaactg-3'or a complementary strand corresponding thereto, a gene amplification primer comprising 5'-catcaagcttctgaagcgtcaga-3' or a complementary strand corresponding thereto, a gene amplification primer comprising 5'-tcaaccaagtggtcgaattgc-3' or a complementary strand corresponding thereto, a gene amplification primer comprising 5'-acggaagatatccarcactaa-3' or a complementary strand corresponding thereto, a gene amplification primer comprising 5'-gcgaacacgatccattgaagtg-3' or a complementary strand corresponding thereto, a gene amplification primer comprising 5'-gatgaacgatttcttcggcatc-3' or a complementary strand corresponding thereto, a gene amplification primer comprising 5'-aaggactttatccagccac-3' or a complementary strand corresponding thereto, a gene amplification primer comprising 5'-ttcttcttgctcacggactttcgc-3' or a complementary strand corresponding thereto, a gene amplification primer comprising 5'-ttctgaattgaacggcggatc-3' or a complementary strand corresponding thereto, and a gene amplification primer comprising 5'-tgtctcttgctcgatcatttgt-3' or a complementary strand corresponding thereto, and gene amplification primers that contain the strands or the complementary strands thereof comprising the above sequences.

### Primers for Vibrio mimicus are as follows.

(1) In the case of *gyrB,* examples of such gene amplification primers include: a gene amplification primer containing a strand or a complementary strand thereof of 15 or more continuous nucleotides of SEQ ID NO: 5 containing 2 or more nucleotides at any of positions 36, 39, 42, 45, 48, or 51 of SEQ ID NO: 5; a gene amplification primer containing a strand or a complementary strand thereof of 15 or more continuous nucleotides of SEQ ID NO: 5 containing 2 or more nucleotides at any of positions 285, 291, or 306 of SEQ ID NO: 5; a gene amplification primer containing a strand or a complementary strand thereof of 15 or more continuous nucleotides of SEQ ID NO: 5 containing 2 or more nucleotides at any of positions 384, 390, or 399 of SEQ ID NO: 5; and a gene amplification primer containing a strand or a complementary strand thereof of 15 or more continuous nucleotides of SEQ ID NO: 5 containing 2 or more nucleotides at any of positions 867, 873, 879, 882, or 885 of SEQ ID NO: 5. More specific examples include a gene amplification primer comprising 5'-ggtagtgaatgccctgtca-3' or a complementary strand corresponding thereto, a gene amplification primer comprising 5'-cggatgagcgtgaagaagataag-3' or a complementary strand corresponding thereto, a gene amplification primer comprising 5'-tgaaaaagtattccacttc-3' or a complementary strand corresponding thereto, a gene amplification primer comprising 5'-gttgaagtggaatactttt-3' or a complementary strand corresponding thereto, and a gene amplification primer comprising 5'-wggcaaaccagckarrtct-3' or a complementary strand corresponding thereto, and gene amplification primers that contain the strands or the complementary strands thereof comprising the above sequences.
(2) In the case of *rpoD,* examples of such gene amplification primers include: a gene amplification primer containing a strand or a complementary strand thereof of 15 or more continuous nucleotides of SEQ ID NO: 6 containing 2 or more nucleotides at any of positions 93, 96, 105, 114, 115, 116, or 117 of SEQ ID NO: 6; a gene amplification primer containing a strand or a complementary strand thereof of 15 or more continuous nucleotides of SEQ ID NO: 6 containing 2 or more nucleotides at any of positions 126, 132, or 141 of SEQ ID NO: 6; a gene amplification primer containing a strand or a complementary strand thereof of 15 or more continuous nucleotides of SEQ ID NO: 6 containing 2 or more nucleotides at any of positions 216, 222, 231, or 240 of SEQ ID NO: 6; a gene amplification primer containing a strand or a complementary strand thereof of 15 or more continuous nucleotides of SEQ ID NO: 6 containing 2 or more nucleotides at any of positions 252, 254, 255, 260, 261, or 264 of SEQ ID NO: 6; a gene amplification primer containing a strand or a complementary strand thereof of 15 or more continuous nucleotides of SEQ ID NO: 6 containing 2 or more nucleotides at any of positions 276, 285, or 291 of SEQ ID NO: 6; a gene amplification primer containing a strand or a complementary strand thereof of 15 or more continuous nucleotides of SEQ ID NO: 6 containing 2 or more nucleotides at any of positions 327, 333, 342, or 345 of SEQ ID NO: 6; a gene amplification primer containing a strand or a complementary strand thereof of 15 or more continuous nucleotides of SEQ ID NO: 6 containing 2 or more nucleotides at any of positions 424, 426, 432, 441, 445, or 446 of SEQ ID NO: 6; a gene amplification primer containing a strand or a complementary strand thereof of 15 or more continuous nucleotides of SEQ ID NO: 6 containing 2 or more nucleotides at any of positions 448, 450, 453, or 468 of SEQ ID NO: 6; a gene amplification primer containing a strand or a complementary strand thereof of 15 or more continuous nucleotides of SEQ ID NO: 6 containing 2 or more nucleotides at any of positions 489, 495, or 501 of SEQ ID NO: 6; a gene amplification primer containing a strand or a complementary strand thereof of 15 or more continuous nucleotides of SEQ ID NO: 6 containing 2 or more nucleotides at any of positions 519, 522, 525, or 540; a gene amplification primer containing a strand or a complementary strand thereof of 15 or more continuous nucleotides of SEQ ID NO: 6 containing 2 or more nucleotides at any of positions 585, 591, 600, 603, or 606 of SEQ ID NO: 6; a gene amplification primer containing a strand or a complementary strand thereof of 15 or more continuous nucleotides of SEQ ID NO: 6 containing 2 or more nucleotides at any of positions 639, 645, 654, or 657 of SEQ ID NO: 6; a gene amplification primer containing a strand or a complementary strand thereof of 15 or more continuous nucleotides of SEQ ID NO: 6 containing 2 or more nucleotides at any of positions 679, 680, 681, 687, or 702 of SEQ ID NO: 6; a gene amplification primer containing a strand or a complementary strand thereof of 15 or more continuous nucleotides of SEQ ID NO: 6 containing 2 or more nucleotides at any of positions 705, 708, 714, 720, or 723 of SEQ ID NO: 6; and a gene amplification primer containing a strand or a complementary strand thereof of 15 or more continuous nucleotides of SEQ ID NO: 6 containing 2 or more nucleotides at any of positions 729, 732, 741, or 750 of SEQ ID NO: 6. More specific examples include a gene amplification primer comprising 5'-cattcttgaacagtttgacaag-3' or a complementary strand corresponding thereto, a gene amplification primer comprising 5'-caggcagaagaactacgtctg-3' or a complementary strand corresponding thereto, a gene amplification primer comprising 5'-agarctctctgaagccgatctcgct-3' or a complementary strand corresponding thereto, a gene amplification primer comprising 5'-gaagatgacgaggtcgcggag-3' or a complementary strand corresponding thereto, a gene amplification primer comprising 5'-gaggatgaagatgaagac-3' or a complementary strand corresponding thereto, a gene amplification primer comprising 5'-gggtattgaccctgagctc-3'or a complementary strand corresponding thereto, a gene amplification primer comprising 5'-taaccaagcatctgaagcttcaag-3'or a complementary strand corresponding thereto, a gene amplification primer comprising 5'-tcaaccaaatggtcaaattgt-3' or a complementary strand corresponding thereto, a gene amplification primer comprising 5'-gcggaaratatccagtaccag-3' or a complementary strand corresponding thereto, a gene amplification primer comprising 5'-acgaacacgatccatcgaggta-3' or a complementary strand corresponding thereto, a gene amplification primer comprising 5'-aataaatgatttctttggcatt-3' or a complementary strand corresponding thereto, a gene amplification primer comprising 5'-gagyactttatcragccat-3' or a complementary strand corresponding thereto, a gene amplification primer comprising 5'-gtcttcttgctcacgtactttttg-3' or a complementary strand corresponding thereto, a gene amplification primer comprising 5'-ttggattgaagggcgaata-3' or a complementary strand corresponding thereto, and a gene amplification primer comprising 5'-agtctcytgttcgatcatctgm-3' or a complementary strand corresponding thereto, and gene amplification primers that contain the strands or the complementary strands thereof comprising the above sequences.

The above regions and strands or complementary strands thereof comprising the above sequences can be directly used as primers. Furthermore, if necessary, other sequences such as adaptor sequences can be contained in primers.

The present invention encompasses a kit for detecting, quantifying, or identifying *Vibrio cholerae* or *mimicus* using these primers and probes in combination with other reagents.

A gene amplification method used in the present invention is not limited to the PCR method. The primers and the probes can be used in a specific amplification method that is based on the specificity of primers or a method for specifically inhibiting amplification. Furthermore, the primers and the probes can also be similarly used in a quantification and amplification reaction using specific amplification primers and a labeled specific probe in combination. Furthermore, a probe sequence can also be used alone. A method for using the probes is not limited to one involving the solid phase or the liquid phase. The primers and the probes can also be utilized for carrying out real-time PCR using a reagent such as cyber green for detecting amplified double-stranded DNA or real-time PCR to which FRET or the like is applied.

The use of information on specificity between phyletic group (Figs. 2, 3, and 4) of the nucleotide sequences obtained in the present invention enables the design of probes having high specificity and gene amplification primers having high specificity and excellent amplification efficiency. Table 2 shows the sequences of PCR primers produced based on specific nucleotide information regarding *Vibrio cholerae* or *Vibrio mimicus* (Figs. 2, 3, and 4) obtained in the present invention.

**Table 2.**

| Primers for specifically detecting *V. cholerae* and *V. mimicus* | | | | | |
|---|---|---|---|---|---|
| Target gene | Primer | Sequence | Length | Position | Direction |
| *gyrB* | CMgF | gaaytctggcgtgtcgatcaag | 22 | 258-279 | Sense |
| | CMgR | catrtagttgttcaaagtacgg | 22 | 564-543 | Antisense |
| *rpoD* | CMrF | gaycctaacgacatggaaacc | 21 | 166-186 | Sense |
| | CMrR | gtcacgaccaaattcattaac | 21 | 420-400 | Antisense |

Information concerning phylogenetic groups has been obtained. Thus, it is possible to design specific primers or probes for almost all of the analyzed sequences.

The present invention will be hereafter described in detail by referring to examples. Each example is an embodiment of the present invention, and the present invention is not limited by these examples.

This specification includes part or all of the contents as disclosed in the specification or drawings of Japanese Patent Application No. 2002-362878, which is a priority document of the present application.

### BRIEF DESCRIPTION OF THE DRAWINGS

Fig. 1 shows the result of molecular phylogenetic analysis after linking partial sequences of the *gyrB* and *rpoD* genes. This is a molecular phylogenetic tree produced by the neighbor-joining method, showing that *Vibrio cholerae* and *mimicus* belong to a mono phyletic group differing from those of other bacteria belonging to the genus *Vibrio.* Furthermore, each of *Vibrio cholerae* and *Vibrio mimicus* forms an independent mono phyletic group.
Fig. 2 shows the results of determination of and comparison between the consensus sequence (upper case) of the *gyrB* gene of the cluster to which *Vibrio cholerae* and *mimicus* belong (shown in Fig. 1) and the consensus sequence (lower case) of the *gyrB* gene of closely related clusters (C1, 2, and 3 in Fig. 1). Sites denoted with "●" are nucleotides specific to *Vibrio cholerae* and *mimicus.*
Fig. 3 shows the results of determination of and comparison between the consensus sequence (upper case) of the *rpoD* gene of the cluster to which *Vibrio cholerae* and *mimicus* belong (shown in Fig. 1) and the consensus sequence (lower case) of the *rpoD* gene of the closely related clusters (C1, 2, and 3 in Fig. 1). Sites denoted with "●" are nucleotides specific to *Vibrio cholerae* and *mimicus.*
Fig. 4 shows the results of determination of and comparison between the consensus sequence (upper case) of the *gyrB* gene of the cluster to which *Vibrio cholerae* belongs (shown in Fig. 1) and the consensus sequence (lower case) of the *gyrB* gene of the cluster to which *Vibrio mimicus* belongs (shown in Fig. 1). Sites denoted with "●" are nucleotides specific to *Vibrio cholerae.*
Fig. 5 shows the results of determination of and comparison between the consensus sequence (upper case) of the *rpoD* gene of the cluster to which *Vibrio cholera* belongs (shown in Fig. 1) and the consensus sequence (lower case) of the *rpoD* gene of the cluster to which *Vibrio mimicus* belongs (shown in Fig. 1). Sites denoted with "●" are nucleotides specific to *Vibrio cholerae.*
Fig. 6 shows the results of determination of and comparison between the consensus sequence (upper case) of the *gyrB* gene of the cluster to which *Vibrio mimicus* belongs (shown in Fig. 1) and the consensus sequence (lower case) of the *gyrB* gene of the cluster to which *Vibrio cholerae* belongs (shown in Fig. 1). Sites denoted with "●" are nucleotides specific to *Vibrio mimicus.*
Fig. 7 shows the results of determination of and comparison between the consensus sequence (upper case) of the *rpoD* gene of the cluster to which *Vibrio mimicus* belongs (shown in Fig. 1) and the consensus sequence (lower case) of the *rpoD* gene of the cluster to which *Vibrio cholerae* belongs (shown in Fig. 1). Sites denoted with "**●"** are nucleotides specific to *Vibrio mimicus.*

### BEST MODE OF CARRYING OUT THE INVENTION

### [Example 1]

An example of using gene amplification primers (shown in Table 2) designed and obtained according to the present invention using regions specific to *Vibrio cholerae* and *mimicus* is shown. In addition, primers described in claim 5 (2) and (3) and claim 11 (2) and (7) correspond to CMgF, CMgR, CMrF, and CMrR in Table 2, respectively. PCR was carried out using chromosomal DNAs as templates extracted from test strains, AmpliTaq Gold (PE Applied Biosystems), and a total of 20 µl of reaction solution. Regarding thermal cycler conditions, after 10 minutes of heating at 95°C, 35 cycles of 94°C for 1 minute, 1 minute of annealing (see Table 5 for annealing temperatures), and 72°C for 1 minute were conducted, finally followed by 10 minutes of elongation reaction at 72°C. Samples obtained after reaction were subjected to 1% agarose gel electrophoresis, and then stained with ethidium bromide. The presence or the absence of amplified genes was confirmed under UV irradiation. Amplified products were confirmed only for the DNAs derived from the bacterial strains determined to belong to *Vibrio cholerae* and *mimicus* with both combinations of CMgF and CMgR targeting the *gyrB* gene and CMrF and CMrR targeting the *rpoD* gene (Table 6).

### [Example 2]

An example of using gene amplification primers (shown in Table 3 and Table 4) designed and obtained according to the present invention using regions specific to *Vibrio cholerae* or *mimicus* is shown. In addition, primers described in claim 19 (1), (3), (4), and (5) and claim 25 (1) and (14) are specific to *Vibrio cholera.* These primers are described in CF1, CF2, CR2, CR1, CrF1, and CrR1 in Table 3. Primers described in claim 33 (1), (3), (4), and (5) and claim 39 (7) and (13) are specific to *Vibrio mimicus.* These primers correspond to MF1, MF2, MR2, MR1, MrF1, and MrR1 in Table 4, respectively.

In a manner similar to Example 1, PCR was carried out using as templates chromosomal DNAs extracted from test strains (see Table 5 for annealing temperatures). In all cases, the use of *Vibrio cholera-specific* primers resulted in detection of amplification products only from *Vibrio cholera* and the use of *Vibrio* mimicus-specific primers resulted in detection of amplification products only from *Vibrio mimicus* (Table 6).

**Table 3.**

| Primers for specifically detecting *V. cholerae* | | | | | |
|---|---|---|---|---|---|
| Target gene | Primer | Sequence | Length | Position | Direction |
| *gyrB* | CF1 | ggtggttaacgcgctytct | 19 | 33-51 | Sense |
| | CR2 | gttaaagtggaagactttc | 19 | 402-384 | Antisense |
| | CF2 | tgagaaagtcttccacttt | 19 | 381-399 | Sense |
| | CR1 | gggtaagccwgcaagatcc | 19 | 885-867 | Antisense |
| *rpoD* | CrF1 | attcttgagcagtttgatcgt | 21 | 97-117 | Sense |
| | CrR1 | ttctgaattgaacggcggatc | 21 | 725-705 | Antisense |

**Table 4.**

| Primers for specifically detecting *V. mimicus* | | | | | |
|---|---|---|---|---|---|
| Target gene | Primer | Sequence | Length | Position | Direction |
| | MF1 | ggtagtgaatgccctgtca | 19 | 33-51 | Sense |
| *gyrB* | | | | | |
| | MR2 | gttgaagtggaatactttt | 19 | 402-384 | Antisense |
| | MF2 | tgaaaaagtattccacttc | 19 | 381-399 | Sense |
| | MR1 | wggcaaaccagckarrtct | 19 | 885-867 | Antisense |
| | MrF1 | taaccaagcatctgaagcttcaag | 24 | 423-446 | Sense |
| *rpoD* | | | | | |
| | MrR1 | gtcttcttgctcacgtactttttg | 24 | 702-679 | Antisense |

**Table 5.**

| PCR conditions for primers for specifically detecting *V. cholerae* and *V. mimicus* | | | | | | |
|---|---|---|---|---|---|---|
| Target gene | Sense primer | Antisense primer | Amplification product (bp) | PCR conditions | | |
| | | | | Annealing temperature (°C) | Number of cycles | Primer temperature (µM) |
| | CMgF | CMgR | 307 | 60 | 35 | 0.1 |
| *gyrB (V. cholerae, V. mimicus)* | | | | | | |
| *rpoD (V*. *cholerae, V mimicus)*. | CMrF | CMrR | 255 | 60 | 35 | 0.1 |
| *gyrB (V. cholerae)* | CF1 | CR2 | 370 | 65 | 35 | 0.1 |
| | CF2 | CR1 | 505 | 60 | 35 | 0.1 |
| *rpoD (V. cholerae)* | CrF1 | CrR1 | 629 | 65 | 35 | 0.1 |
| *gyrB* (V. *mimicus)* | MF1 | MR2 | 370 | 65 | 35 | 0.1 |
| | MF2 | MR1 | 505 | 60 | 35 | 0.1 |
| *rpoD (V. mimicus)* | MrF1 | MrR1 | 280 | 65 | 35 | 0.1 |

**Table 6**

| PCR | | | | | | | | | | | | |
|---|---|---|---|---|---|---|---|---|---|---|---|---|
| Serial No. | Name | Strain name | Serotype | Cholerae toxin gene | CMgF & CMgR | CMrF & CMrR | CF1 & CR2 | CF2 & CR1 | CrF1 & CrR1 | MF1 & MR2 | MF2 & MR1 | MrF1 & MrR1 |
| 1 | *V. vulnificus* | ATCC 27562 T | | | - | - | - | - | - | - | - | - |
| 2 | *V. vulnificus* | ATCC 29306 | | | - | - | - | - | - | - | - | - |
| 3 | *V. vulnificus* | ATCC 29307 | | | - | - | - | - | - | - | - | - |
| 4 | *V. vulnificus* | ATCC 33147 | | | - | - | - | - | - | - | - | - |
| *5* | *V. vulnificus* | ATCC 33148 | | | - | - | - | - | - | - | - | - |
| 6 | *V. vulnificus* | ATCC 33149 | | | - | - | - | - | - | - | - | - |
| 7 | *V. vulnificus* | ATCC 33814 | | | - | - | - | - | - | - | - | - |
| 8 | *V. vulnificus* | ATCC 33815 | | | - | - | - | - | - | - | - | - |
| 9 | *V. vulnificus* | ATCC 33816 | | | - | - | - | - | - | - | - | - |
| 10 | *V. vulnificus* | ATCC 33817 | | | - | - | - | - | - | - | - | - |
| 11 | *V. vulnificus* | ATCC 43382 | | | - | - | - | - | - | - | - | - |
| 12 | *V. vulnificus* | ATCC BAA-86 | | | - | - | - | - | - | - | - | - |
| 13 | *V. vulnificus* | ATCC BAA-87 | | | - | - | - | - | - | - | - | - |
| 14 | *V. vulnificus* | ATCC BAA-88 | | | - | - | - | - | - | - | - | - |
| 15 | *V. vulnificus* | ATCC BAA-89 | | | - | - | - | - | - | - | - | - |
| 16 | *V. vulnificus* | ATCC BAA-90 | | | - | - | - | - | - | - | - | - |
| 17 | *V. vulnificus* | JCM 3726 | | | - | - | - | - | - | - | - | - |
| 18 | *V. vulnificus* | JCM 3727 | | | - | - | - | - | - | - | - | - |
| 19 | *V. vulnificus* | JCM 3728 | | | - | - | - | - | - | - | - | - |
| 20 | *V. vulnificus* | JCM 3729 | | | - | - | - | - | - | - | - | - |
| 21 | *V. vulnificus* | JCM 3730 | | | - | - | - | - | - | - | - | - |
| 22 | *V. vulnificus* | JCM 3731 | | | - | - | - | - | - | - | - | - |
| 23 | *V. vulnificus* | No. 4 | | | - | - | - | - | - | - | - | - |
| 24 | *V vulnificus* | No. 9 | | | - | - | - | - | - | - | - | - |
| 25 | *V. vulnificus* | No. 66 | | | - | - | - | - | - | - | - | - |
| 26 | *V. vulnificus* | No. 74 | | | - | - | - | - | - | - | - | - |
| 27 | *V. vulnificus* | No. 81 | | | - | - | - | - | - | - | - | - |
| 28 | *V. vulnificus* | No. 130 | | | - | - | - | - | - | - | - | - |
| 29 | *V. vulnificus* | No. 196 | | | - | - | - | - | - | - | - | - |
| 30 | *V. vulnificus* | No. 202 | | | - | - | - | - | - | - | - | - |
| 31 | *V. vulnificus* | No. 496 | | | - | - | - | - | - | - | - | - |
| 32 | *V. vulnificus* | No. 965 | | | - | - | - | - | - | - | - | - |
| 33 | *V. cholerae* | DU1 | monO1 | + | + | + | + | + | + | - | - | - |
| *34* | *V. cholerae* | DU2 | O1 | + | + | + | + | + | + | - | - | - |
| 35 | *V. cholerae* | DU3 | O1 | + | + | + | + | + | + | - | - | - |
| *36* | *V. cholerae* | DU6 | O1 | + | + | + | + | + | + | - | - | - |
| 37 | *V. cholerae* | DU19 | O1 | + | + | + | + | + | + | - | - | - |
| 38 | *V. cholerae* | DU63 | monO1 | + | + | + | + | + | + | - | - | - |
| 39 | *V. cholerae* | DU81 | O1 | + | + | + | + | + | + | - | - | - |
| 40 | *V. cholerae* | DU94 | O1 | + | + | + | + | + | + | - | - | - |
| 41 | *V. cholerae* | T2 | monO1 | + | + | + | + | + | + | - | - | - |
| 42 | *V. cholerae* | T6 | monO1 | + | + | + | + | + | + | - | - | - |
| 43 | *V. cholerae* | T97 | monO1 | + | + | + | + | + | + | - | - | - |
| 44 | *V. cholerae* | T98 | O1 | + | + | + | + | + | + | - | - | - |
| 45 | *V. cholerae* | T116 | monO1 | + | + | + | + | + | + | - | - | - |
| 46 | *V. cholerae* | NM20 | monO1 | + | + | + | + | + | + | - | - | - |
| 47 | *V. cholerae* | NM26 | O1 | + | + | + | + | + | + | - | - | - |
| 48 | *V. cholerae* | NM48 | O1 | + | + | + | + | + | + | - | - | - |
| 49 | *V. cholerae* | NM67 | monO1 | + | + | + | + | + | + | - | - | - |
| 50 | *V. cholerae* | NM84 | monO1 | + | + | + | + | + | + | - | - | - |
| 51 | *V. cholerae* | NM85 | monO1 | + | + | + | + | + | + | - | - | - |
| 52 | *V. cholerae* | Q37 | O1 | + | + | + | + | + | + | - | - | - |
| 53 | *V. cholerae* | Q57 | O1 | + | + | + | + | + | + | - | - | - |
| 54 | *V. cholerae* | Q59 | monO1 | + | + | + | + | + | + | - | - | - |
| 55 | *V. cholerae* | Q66 | monO1 | + | + | + | + | + | + | - | - | - |
| 56 | *V. cholerae* | Q70 | monO1 | + | + + | + | + | + | + | - | - | - |
| 57 | *V. cholerae* | Q90 | monO1 | + | + | + | + | + | + | - | - | - |
| 58 | *V. cholerae* | K47 | monO1 | + | + | + | + | + | + | - | - | - |
| 59 | *V. cholerae* | OPC24 | monO1 | + | + | + | + | + | + | - | - | - |
| 60 | *V. cholerae* | OPC39 | monO1 | + | + | + | + | + | + | - | - | - |
| 61 | *V. cholerae* | OPC60 | O1 | + | + | + | + | + | + | - | - | - |
| 62 | *V. mimicus* | ATCC 33563 T | | - | + | + | - | - | - | + | + | + |
| 63 | *V. mimicus* | ATCC 33654 | | - | + | + | - | - | - | + | + | + |
| 64 | *V. mimicus* | ATCC 33655 | | + | + | + | - | - | - | + | + | + |
| 65 | *V. mimicus* | ATCC 700326 | | + | + | + | - | - | - | + | + | + |
| 66 | *V. diazo- Trophicus* | ATCC 33466 T | | | - | - | - | - | - | - | - | - |
| 67 | V. navarrensis | ATCC 51183 T | | | - | - | - | - | - | - | - | - |
| 68 | *V. metschnikovii* | ATCC 700040 T | | | - | - | - | - | - | - | - | - |
| 69 | *V. cincinnatiensis* | ATCC 35912 T | | | - | - | - | - | - | - | - | - |
| 70 | *V. ordalii* | NCIMB 2167 T | | | - | - | - | - | - | - | - | - |
| 71 | *Listonella anguillarum* | NCIMB 6 T | | | - | - | - | - | - | - | - | - |
| 72 | *V. alginolyticus* | IFO 15630 T | | | - | - | - | - | - | - | - | - |
| 73 | *V. campbellii* | IFO 15631 T | | | - | - | - | - | - | - | - | - |
| 74 | *V. carchariae* | IFO 15632 T | | | - | - | - | - | - | - | - | - |
| 75 | *V. harveyi* | IFO 15634 T | | | - | - | - | - | - | - | - | - |
| *76* | *V. nereis* | IFO 15637 T | | | - | - | - | - | - | - | - | - |
| 77 | *V. parahaemolyticus* | IFO IFO 12711 T | | | - | - | - | - | - | - | - | - |
| 78 | *V. proteolyticus* | IFO 13287 T | | | - | - | - | - | - | - | - | - |
| 79 | *V. tubiashii* | IFO 15644 T | | | - | - | - | - | - | - | - | - |
| 80 | *80 Vibrio sp.* | V11 | | | - | - | - | - | - | - | - | - |
| 81 | *Vibrio sp.* | V41 | | | - | - | - | - | - | - | - | - |
| 82 | *Shewanella sp.* V52 | | | | - | - | - | - | - | - | - | - |
| 83 . | *Vibrio* sp | V65 | | | - | - | - | - | - | - | - | - |
| 84 | *Vibrio sp.* | V70 | | | - | - | - | - | - | - | - | - |
| 85 | *V. hollisae* | ATCC 33564 T | | | - | - | - | - | - | - | - | - |

All publications, patents and patent applications cited herein are incorporated herein by reference in their entirety.

### Industrial Applicability

The *gyrB* and *rpoD* gene primers and probes of the present invention have been designed based on the understanding of the phylogenetic relationship of *Vibrio cholerae* and *Vibrio mimicus.* Thus, the primers and the probes have been studied for improving specificity and are excellent in terms of detection accuracy. Hence, the primers and the probes are advantageous when direct detection is carried out under circumstances where a bacterium is not isolated from foods, clinical specimens, or the like that are contaminated with closely related bacterial species.

## Claims

1. A fragment of a gene represented by SEQ ID NO: 1, which is a fragment of a gene *(gyrB)* encoding a DNA gyrase β subunit of SEQ ID NO: 1 in the sequence listing and contains one or more nucleotides at any of positions (also referred to as nucleotide numbers) 21, 96, 107, 126, 153, 190, 258, 270, 279, 285, 357, 543, 552, 557, 600, 690, 702, 714, 729, 733, 734, 759, 771, 782, 786, 792, 795, or 885, said positions being unique to *Vibrio cholerae* and *Vibrio mimicus* bacterial groups, where said gene fragment can be used for designing a specific gene amplification primer or probe.

2. A gene amplification primer, which contains a strand or a complementary strand thereof containing 15 or more continuous nucleotides, where one or more nucleotides are at any of positions 21, 96, 107, 126, 153, 190, 258, 270, 279, 285, 357, 543, 552, 557, 600, 690, 702, 714, 729, 733, 734, 759, 771, 782, 786, 792, 795, or 885 in the gene *(gyrB)* encoding the DNA gyrase β subunit of SEQ ID NO: 1 in the sequence listing containing the fragment of claim 1, said positions being unique to the *Vibrio cholerae* and *Vibrio mimicus* bacterial groups.

3. The gene amplification primer of claim 2, wherein a region containing at a high frequency two or more positions unique to the *Vibrio cholerae* and *Vibrio mimicus* bacterial groups as specified in claim 1 is used.

4. The gene amplification primer of claim 2, wherein the 3' terminal nucleotide is a nucleotide at a position that is unique to the *Vibrio cholerae* and *Vibrio mimicus* bacterial groups as specified in claim 1.

5. The gene amplification primer of claim 2, which contains any one of:
(1) 5'-tycaywcscaaacttacca-3' or a complementary strand corresponding thereto;
(2) 5'-gaaytctggcgtgtcgatcaag-3' or a complementary strand corresponding thereto;
(3) 5'-catrtagttgttcaaagtacgg-3' or a complementary strand corresponding thereto;
(4) 5'-ggatttyacytccgaagaaacyagc-3' or a complementary strand corresponding thereto;
(5) 5'-ygccagcttctcattcatr-3' or a complementary strand corresponding thereto;
(6) 5'-cgcttcgcttgggttttcc-3' or a complementary strand corresponding thereto; or
(7) 5'-caataatcttcgaacaaacgt-3' or a complementary strand corresponding thereto.

6. A probe for detecting, quantifying, or identifying *Vibrio cholerae* and *Vibrio mimicus,* which contains a strand or a complementary strand thereof containing 15 or more continuous nucleotides, where one or more nucleotides are at any of positions 21, 96, 107, 126, 153, 190, 258, 270, 279, 285, 357, 543, 552, 557, 600, 690, 702, 714, 729, 733, 734, 759, 771, 782, 786, 792, 795, or 885 in the gene *(gyrB)* encoding the DNA gyrase β subunit of SEQ ID NO: 1 in the sequence listing, said positions being unique to the *Vibrio cholerae* and *Vibrio mimicus* bacterial groups.

7. A fragment of a gene represented by SEQ ID NO: 2 in the sequence listing, which contains one or more nucleotides at any of positions 3, 27, 66, 67, 75, 90, 117, 123, 141, 144, 177, 178, 180, 186, 223, 227, 228, 231, 250, 251, 255, 257, 259, 264, 300, 301, 302, 303, 305, 313, 314, 350, 351, 362, 369, 373, 374, 380, 390, 400, 402, 409, 410, 415, 416, 423, 427, 433, 444, 447, 504, 510, 513, 543, 556, 558, 618, 638, 649, 663, 685, 711, 747, 757, 762, 763, or 789 in a gene *(rpoD)* encoding an RNA polymerase σ70 factor of SEQ ID NO: 2, said positions being unique to the *Vibrio cholerae* and *Vibrio mimicus* bacterial groups, where said gene fragment can be used for designing a specific gene amplification primer or probe.

8. A gene amplification primer, which contains a strand or a complementary strand thereof containing 15 or more continuous nucleotides of SEQ ID NO: 2 in the sequence listing, where one or more nucleotides are at any of positions 3, 27, 66, 67, 75, 90, 117, 123, 141, 144, 177, 178, 180, 186, 223, 227, 228, 231, 250, 251, 255, 257, 259, 264, 300, 301, 302, 303, 305, 313, 314, 350, 351, 362, 369, 373, 374, 380, 390, 400, 402, 409, 410, 415, 416, 423, 427, 433, 444, 447, 504, 510, 513, 543, 556, 558, 618, 638, 649, 663, 685, 711, 747, 757, 762, 763, or 789 in the gene *(rpoD)* encoding the RNA polymerase σ70 factor of SEQ ID NO: 2, said positions being unique to the *Vibrio cholerae* and *Vibrio mimicus* bacterial groups.

9. The gene amplification primer of claim 8, wherein a region containing at a high frequency two or more positions unique to the *Vibrio cholerae* and *Vibrio mimicus* bacterial groups as specified in claim 8 is used.

10. The gene amplification primer of claim 8, wherein the 3' terminal nucleotide is a nucleotide at a position that is unique to the *Vibrio cholerae* and *Vibrio mimicus* bacterial groups as specified in claim 8.

11. The gene amplification primer of claim 8, which contains any one of:
(1) 5'-gattgctgagtatcctggaaccatc-3' or a complementary strand corresponding thereto;
(2) 5'-gaycctaacgacatggaaacc-3' or a complementary strand corresponding thereto;
(3) 5'-ttcwgarctytctgaagcs-3' or a complementary strand corresponding thereto;
(4) 5'-agatgaygmkgtcgysgar-3' or a complementary strand corresponding thereto;
(5) 5'-cgacggtgaaagyagcgacag-3' or a complementary strand corresponding thereto;
(6) 5'-caatgaactgcgcggyaagtt-3' or a complementary strand corresponding thereto;
(7) 5'-gtcacgaccaaattcattaac-3' or a complementary strand corresponding thereto;
(8) 5'-gyytgamgcttcagawgcttgrtka-3' or a complementary strand corresponding thereto;
(9) 5'-ygargtrcgcagagtttcaacc-3' or a complementary strand corresponding thereto;
(10) 5'-catyaccaarcgytcttgg-3' or a complementary strand corresponding thereto; or
(11) 5'-cgytcaacagacagtgawgtc-3' or a complementary strand corresponding thereto.

12. A probe for detecting, quantifying, or identifying the *Vibrio cholerae* and *Vibrio mimicus* bacterial groups, which contains a strand or a complementary strand thereof containing 15 or more continuous nucleotides of SEQ ID NO: 2 in the sequence listing, where one or more nucleotides are at any of positions 3, 27, 66, 67, 75, 90, 117, 123, 141, 144, 177, 178, 180, 186, 223, 227, 228, 231, 250, 251, 255, 257, 259, 264, 300, 301, 302, 303, 305, 313, 314, 350, 351, 362, 369, 373, 374, 380, 390, 400, 402, 409, 410, 415, 416, 423, 427, 433, 444, 447, 504, 510, 513, 543, 556, 558, 618, 638, 649, 663, 685, 711, 747, 757, 762, 763, or 789 in the gene *(rpoD)* encoding the RNA polymerase σ70 factor of SEQ ID NO: 2, said positions being unique to the *Vibrio cholerae* and *Vibrio mimicus* bacterial groups.

13. A method for detecting, quantifying, or identifying the *Vibrio cholerae* and *Vibrio mimicus* bacterial groups, wherein the primer or the probe of any one of claims 2 to 6 or 8 to 12 is used.

14. A kit for detecting, quantifying, or identifying the *Vibrio cholerae* and *Vibrio mimicus* bacterial groups, wherein the primer or the probe of any one of claims 2 to 6 or 8 to 12 is used.

15. A fragment of a gene represented by SEQ ID NO: 3 in the sequence listing, which contains one or more nucleotides at any of positions 15, 36, 39, 42, 45, 48, 51, 90, 111, 133, 226, 285, 291, 306, 330, 384, 390, 399, 507, 708, 756, 837, 867, 873, 879, 882, or 885 in a gene *(gyrB)* encoding a DNA gyrase β subunit of SEQ ID NO: 3, said positions being unique to the *Vibrio cholerae* bacterial group, where said gene fragment can be used for designing a specific gene amplification primer or probe.

16. A gene amplification primer, which contains a strand or a complementary strand thereof containing 15 or more continuous nucleotides of SEQ ID NO: 3 in the sequence listing, where one or more nucleotides are at any of positions 15, 36, 39, 42, 45, 48, 51, 90, 111, 133, 226, 285, 291, 306, 330, 384, 390, 399, 507, 708, 756, 837, 867, 873, 879, 882, or 885 in the gene *(gyrB)* encoding the DNA gyrase β subunit of SEQ ID NO: 3, said positions being unique to the *Vibrio cholerae* bacterial group.

17. The gene amplification primer of claim 16, wherein the 3' terminal nucleotide is a nucleotide at a position that is unique to the *Vibrio cholerae* bacterial group as specified in claim 16.

18. The gene amplification primer of claim 16, wherein a region containing at a high frequency two or more positions unique to the *Vibrio cholerae* bacterial group as specified in claim 16 is used.

19. The gene amplification primer of claim 16, which contains any one of:
(1) 5'-ggtggttaacgcgctytct-3' or a complementary strand corresponding thereto;
(2) 5'-ycgatgaacgtgaagaagataaa-3' or a complementary strand corresponding thereto;
(3) 5'-tgagaaagtcttccacttt-3' or a complementary strand corresponding thereto;
(4) 5'-gttaaagtggaagactttc-3' or a complementary strand corresponding thereto; or
(5) 5'-gggtaagccwgcaagatcc-3' or a complementary strand corresponding thereto.

20. A probe for detecting, quantifying, or identifying the *Vibrio cholerae* bacterial group, which contains a strand or a complementary strand thereof containing 15 or more continuous nucleotides, where one or more nucleotides are at any of positions 15, 36, 39, 42, 45, 48, 51, 90, 111, 133, 226, 285, 291, 306, 330, 384, 390, 399, 507, 708, 756, 837, 867, 873, 879, 882, or 885 in the gene *(gyrB)* encoding the DNA gyrase β subunit of SEQ ID NO: 3 in the sequence listing, said positions being unique to the *Vibrio cholerae* bacterial group.

21. A fragment of a gene represented by SEQ ID NO: 4 in the sequence listing, which contains one or more nucleotides at any of positions 12, 93, 96, 105, 114, 115, 116, 117, 126, 132, 141, 156, 198, 201, 216, 222, 231, 240, 252, 254, 255, 260, 261, 264, 276, 285, 291, 327, 333, 342, 345, 424, 426, 432, 441, 445, 446, 448, 450, 453, 468, 489, 495, 501, 519, 522, 525, 540, 549, 570, 585, 591, 600, 603, 606, 639, 645, 654, 657, 666, 675, 679, 680, 681, 687, 702, 705, 708, 714, 720, 723, 729, 732, 741, 750, 765, 768, 795, or 804 in a gene *(rpoD)* encoding an RNA polymerase σ70 factor of SEQ ID NO: 4, said positions being unique to the *Vibrio cholerae* bacterial group, where said gene fragment can be used for designing a specific gene amplification primer or probe.

22. A gene amplification primer, which contains a strand or a complementary strand thereof containing 15 or more continuous nucleotides of SEQ ID NO: 4 in the sequence listing, where one or more nucleotides are at any of positions 12, 93, 96, 105, 114, 115, 116, 117, 126, 132, 141, 156, 198, 201, 216, 222, 231, 240, 252, 254, 255, 260, 261, 264, 276, 285, 291, 327, 333, 342, 345, 424, 426, 432, 441, 445, 446, 448, 450, 453, 468, 489, 495, 501, 519, 522, 525, 540, 549, 570, 585, 591, 600, 603, 606, 639, 645, 654, 657, 666, 675, 679, 680, 681, 687, 702, 705, 708, 714, 720, 723, 729, 732, 741, 750, 765, 768, 795, or 804 in the gene *(rpoD)* encoding the RNA polymerase σ70 factor of SEQ ID NO: 4, said positions being unique to the *Vibrio cholerae* bacterial group.

23. The gene amplification primer of claim 22, wherein the 3' terminal nucleotide is a nucleotide at a position that is unique to the *Vibrio cholerae* bacterial group as specified in claim 22.

24. The gene amplification primer of claim 22, wherein a region containing at a high frequency two or more positions unique to the *Vibrio cholerae* bacterial group as specified in claim 22 is used.

25. The gene amplification primer of claim 22, which contains any one of:
(1) 5'-attcttgagcagtttgatcgt-3' or a complementary strand corresponding thereto;
(2) 5'-caggccgaagagctacgtctc-3' or a complementary strand corresponding thereto;
(3) 5'-tgagctttctgaagcggatctcgcg-3' or a complementary strand corresponding thereto;
(4) 5'-gaagatgatgctgtcgtcgaa-3' or a complementary strand corresponding thereto;
(5) 5'-gaagatgaagacgaagat-3' or a complementary strand corresponding thereto;
(6) 5'-cggtatcgaccctgaactg-3' or a complementary strand corresponding thereto;
(7) 5'-catcaagcttctgaagcgtcaga-3' or a complementary strand corresponding thereto;
(8) 5'-acggaagatatccarcactaa-3' or a complementary strand corresponding thereto;
(9) 5'-tcaaccaagtggtcgaattgc-3' or a complementary strand corresponding thereto;
(10) 5'-gcgaacacgatccattgaagtg-3' or a complementary strand corresponding thereto;
(11) 5'-gatgaacgatttcttcggcatc-3' or a complementary strand corresponding thereto;
(12) 5'-aaggactttatccagccac-3' or a complementary strand corresponding thereto;
(13) 5'-ttcttcttgctcacggactttcgc-3' or a complementary strand corresponding thereto;
(14) 5'-ttctgaattgaacggcggatc-3' or a complementary strand corresponding thereto; or
(15) 5'-tgtctcttgctcgatcatttgt-3' or a complementary strand corresponding thereto.

26. A probe for detecting, quantifying, or identifying the *Vibrio cholerae* bacterial group, which contains a strand or a complementary strand thereof containing 15 or more continuous nucleotides of SEQ ID NO: 4 in the sequence listing, where one or more nucleotides are at any of positions 12, 93, 96, 105, 114, 115, 116, 117, 126, 132, 141, 156, 198, 201, 216, 222, 231, 240, 252, 254, 255, 260, 261, 264, 276, 285, 291, 327, 333, 342, 345, 424, 426, 432, 441, 445, 446, 448, 450, 453, 468, 489, 495, 501, 519, 522, 525, 540, 549, 570, 585, 591, 600, 603, 606, 639, 645, 654, 657, 666, 675, 679, 680, 681, 687, 702, 705, 708, 714, 720, 723, 729, 732, 741, 750, 765, 768, 795, or 804 in the gene *(rpoD)* encoding the RNA polymerase σ70 factor of SEQ ID NO: 4, said positions being unique to the *Vibrio cholerae* bacterial group.

27. A method for detecting, quantifying, or identifying the *Vibrio cholerae* bacterial group, wherein the primer or the probe of any one of claims 16 to 20 or 22 to 26 is used.

28. A kit for detecting, quantifying, or identifying the *Vibrio cholerae* bacterial group, wherein the primer or the probe of any one of claims 16 to 20 or 22 to 26 is used.

29. A fragment of a gene represented by SEQ ID NO: 5 in the sequence listing, which contains one or more nucleotides at any of positions 15, 36, 39, 42, 45, 48, 51, 90, 111, 133, 226, 285, 291, 306, 330, 384, 390, 399, 507, 708, 756, 837, 867, 873, 879, 882, or 885 in a gene *(gyrB)* encoding a DNA gyrase β subunit of SEQ ID NO: 5, said positions being unique to the *Vibrio mimicus* bacterial group, where said gene fragment can be used for designing a specific gene amplification primer or probe.

30. A gene amplification primer, which contains a strand or a complementary strand thereof containing 15 or more continuous nucleotides of SEQ ID NO: 3 in the sequence listing, where one or more nucleotides are at any of positions 15, 36, 39, 42, 45, 48, 51, 90, 111, 133, 226, 285, 291, 306, 330, 384, 390, 399, 507, 708, 756, 837, 867, 873, 879, 882, or 885 in the gene *(gyrB)* encoding the DNA gyrase β subunit of SEQ ID NO: 3, said positions being unique to the *Vibrio mimicus* bacterial group.

31. The gene amplification primer of claim 30, wherein a region containing at a high frequency two or more positions unique to the *Vibrio mimicus* bacterial group as specified in claim 30 is used.

32. The gene amplification primer of claim 30, wherein the 3' terminal nucleotide is a nucleotide at a position that is unique to the *Vibrio mimicus* bacterial group as specified in claim 31.

33. The gene amplification primer of claim 30, which contains any one of:
(1) 5'-ggtagtgaatgccctgtca-3' or a complementary strand corresponding thereto;
(2) 5'-cggatgagcgtgaagaagataag-3' or a complementary strand corresponding thereto;
(3) 5'-tgaaaaagtattccacttc-3' or a complementary strand corresponding thereto;
(4) 5'-gttgaagtggaatactttt-3' or a complementary strand corresponding thereto; or
(5) 5'-wggcaaaccagckarrtct-3' or a complementary strand corresponding thereto.

34. A probe for detecting, quantifying, or identifying the *Vibrio mimicus* bacterial group, which contains a strand or a complementary strand thereof containing 15 or more continuous nucleotides of SEQ ID NO: 5 in the sequence listing, where one or more nucleotides are at any of positions 15, 36, 39, 42, 45, 48, 51, 90, 111, 133, 226, 285, 291, 306, 330, 384, 390, 399, 507, 708, 756, 837, 867, 873, 879, 882, or 885 in the gene *(gyrB)* encoding the DNA gyrase β subunit of SEQ ID NO: 5, said positions being unique to the *Vibrio mimicus* bacterial group.

35. A fragment of a gene represented by SEQ ID NO: 6 in the sequence listing, which contains one or more nucleotides at any of positions 12, 93, 96, 105, 114, 115, 116, 117, 126, 132, 141, 156, 198, 201, 216, 222, 231, 240, 252, 254, 255, 260, 261, 264, 276, 285, 291, 327, 333, 342, 345, 424, 426, 432, 441, 445, 446, 448, 450, 453, 468, 489, 495, 501, 519, 522, 525, 540, 549, 570, 585, 591, 600, 603, 606, 639, 645, 654, 657, 666, 675, 679, 680, 681, 687, 702, 705, 708, 714, 720, 723, 729, 732, 741, 750, 765, 768, 795, or 804 in a gene *(rpoD)* encoding an RNA polymerase σ70 factor of SEQ ID NO: 6, said positions being unique to the *Vibrio mimicus* bacterial group, where said gene fragment can be used for designing a specific gene amplification primer or probe.

36. A gene amplification primer, which contains a strand or a complementary strand thereof containing 15 or more continuous nucleotides of SEQ ID NO: 6 in the sequence listing, where one or more nucleotides are at any of positions 12, 93, 96, 105, 114, 115, 116, 117, 126, 132, 141, 156, 198, 201, 216, 222, 231, 240, 252, 254, 255, 260, 261, 264, 276, 285, 291, 327, 333, 342, 345, 424, 426, 432, 441, 445, 446, 448, 450, 453, 468, 489, 495, 501, 519, 522, 525, 540, 549, 570, 585, 591, 600, 603, 606, 639, 645, 654, 657, 666, 675, 679, 680, 681, 687, 702, 705, 708, 714, 720, 723, 729, 732, 741, 750, 765, 768, 795, or 804 in the gene *(rpoD)* encoding the RNA polymerase σ70 factor of SEQ ID NO: 6, said positions being unique to the *Vibrio mimicus* bacterial group.

37. The gene amplification primer of claim 36, wherein a region containing at a high frequency two or more positions unique to the *Vibrio mimicus* bacterial group as specified in claim 36 is used.

38. The gene amplification primer of claim 36, wherein the 3' terminal nucleotide is a nucleotide at a position that is unique to the *Vibrio mimicus* bacterial group as specified in claim 36.

39. The gene amplification primer of claim 36, which contains any one of:
(1) 5'-cattcttgaacagtttgacaag-3' or a complementary strand corresponding thereto;
(2) 5'-caggcagaagaactacgtctg-3' or a complementary strand corresponding thereto;
(3) 5'-agarctctctgaagccgatctcgct-3' or a complementary strand corresponding thereto;
(4) 5'-gaagatgacgaggtcgcggag-3' or a complementary strand corresponding thereto;
(5) 5'-gaggatgaagatgaagac-3' or a complementary strand corresponding thereto;
(6) 5'-gggtattgaccctgagctc-3' or a complementary strand corresponding thereto;
(7) 5'-taacaaagcatctgaagcttcaag-3' or a complementary strand corresponding thereto;
(8) 5'-gcggaaratatccagtaccag-3' or a complementary strand corresponding thereto;
(9) 5'-tcaaccaaatggtcaaattgt-3' or a complementary strand corresponding thereto;
(10) 5'-acgaacacgatccatcgaggta-3' or a complementary strand corresponding thereto;
(11) 5'-aataaatgatttctttggcatt-3' or a complementary strand corresponding thereto;
(12) 5'-gagyactttatcragccat-3' or a complementary strand corresponding thereto;
(13) 5'-gtcttcttgctcacgtactttttg-3' or a complementary strand corresponding thereto;
(14) 5'-ttggattgaagggcgaata-3' or a complementary strand corresponding thereto; or
(15) 5'-agtctcytgttcgatcatctgm-3' or a complementary strand corresponding thereto.

40. A probe for detecting, quantifying, or identifying the *Vibrio mimicus* bacterial group, which contains a strand or a complementary strand thereof containing 15 or more continuous nucleotides of SEQ ID NO: 6 in the sequence listing, where one or more nucleotides are at any of positions 12, 93, 96, 105, 114, 115, 116, 117, 126, 132, 141, 156, 198, 201, 216, 222, 231, 240, 252, 254, 255, 260, 261, 264, 276, 285, 291, 327, 333, 342, 345, 424, 426, 432, 441, 445, 446, 448, 450, 453, 468, 489, 495, 501, 519, 522, 525, 540, 549, 570, 585, 591, 600, 603, 606, 639, 645, 654, 657, 666, 675, 679, 680, 681, 687, 702, 705, 708, 714, 720, 723, 729, 732, 741, 750, 765, 768, 795, or 804 in the gene *(rpoD)* encoding the RNA polymerase σ70 factor of SEQ ID NO: 6 in the sequence listing, said positions being unique to the *Vibrio mimicus* bacterial group.

41. A method for detecting, quantifying, or identifying the *Vibrio mimicus* bacterial group, wherein the primer or the probe of any one of claims 30 to 34 or 36 to 40 is used.

42. A kit for detecting, quantifying, or identifying the *Vibrio mimicus* bacterial group, wherein the primer or the probe of any one of claims 30 to 34 or 36 to 40 is used.
